(19) Europäisches Patentamt
European Patent Office
Office européen des brevets

(11) **EP 2 351 577 A1**

(12) **EUROPEAN PATENT APPLICATION**

(43) Date of publication:
**03.08.2011 Bulletin 2011/31**

(51) Int Cl.:
***A61K 39/00*** *(2006.01)*

(21) Application number: **11155219.6**

(22) Date of filing: **29.12.2005**

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.12.2004 US 640727 P**

(62) Document number(s) of the earlier application(s) in accordance with Art. 76 EPC:
**05855760.4 / 1 838 338**

(71) Applicant: **Mannkind Corporation**
**Valencia,**
**California 91355 (US)**

(72) Inventors:
• **Kundig, Thomas**
**8303, Baserssdorf (CH)**
• **BOT, Adrian, Ion**
**Valencia, CA 91354 (US)**

(74) Representative: **Lasar, Andrea Gisela**
**Maiwald Patentanwalts GmbH**
**Elisenhof, Elisenstrasse 3**
**80335 München (DE)**

Remarks:
This application was filed on 21-02-2011 as a divisional application to the application mentioned under INID code 62.

(54) **Methods to trigger, maintain and manipulate immune responses by targeted administration of biological response modifiers into lymphoid organs**

(57) The present invention includes use of a biological response modifier (BRM) to modulate an immune response in a subject while avoiding or limiting common side-effects associate with BRM use. The BRMs of the present invention can be injected into a secondary lymphatic organ of the subject or into an area of relatively high drainage into a secondary lymphatic organ of the subject. The modulated immune response can be general or antigen-specific.

EP 2 351 577 A1

SEQUENCE LISTING

<110> Kundig, Thomas
      Bot, Adrian Ion

<120> METHODS TO TRIGGER, MAINTAIN AND
      MANIPULATE IMMUNE RESPONSES BY TARGETED ADMINISTRATION OF
      BIOLOGICAL RESPONSE MODIFIERS INTO LYMPHOID ORGANS

<130> MANNK.046VPC

<150> 60/640,727
<151> 2004-12-29

<160> 11

<170> FastSEQ for Windows Version 4.0

<210> 1
<211> 21
<212> DNA
<213> Artificial Sequence

<220>
<223> Synthetically prepared nucleic acid sequence

<400> 1
tccatgacgt tcctgaataa t                                              21

<210> 2
<211> 8
<212> PRT
<213> Artificial Sequence

<220>
<223> Synthetically prepared peptide sequence

<400> 2
Ser Ile Ile Asn Phe Glu Lys Leu
 1               5

<210> 3
<211> 10
<212> PRT
<213> Artificial Sequence

<220>
<223> Synthetically prepared peptide sequence

<400> 3
Glu Leu Ala Gly Ile Gly Ile Leu Thr Val
 1               5                   10

<210> 4
<211> 20
<212> DNA
<213> Artificial Sequence

<220>
<223> Synthetically prepared nucleic acid sequence

<400> 4
tccatgacgt tcctgacgtt                                                20

```
<210> 5
<211> 10
<212> PRT
<213> Artificial Sequence

<220>
<223> Synthetically prepared peptide sequence

<400> 5
Gly Leu Pro Ser Ile Pro Val His Pro Ile
1               5               10


<210> 6
<211> 10
<212> PRT
<213> Artificial Sequence

<220>
<223> Synthetically prepared peptide sequence

<400> 6
Arg Gln Ile Tyr Val Ala Ala Phe Thr Val
1               5               10


<210> 7
<211> 9
<212> PRT
<213> Artificial Sequence

<220>
<223> Synthetically prepared peptide sequence

<400> 7
Ser Leu Leu Gln His Leu Ile Gly Leu
1               5


<210> 8
<211> 10
<212> PRT
<213> Artificial Sequence

<220>
<223> Synthetically prepared peptide sequence

<400> 8
Ala Leu Tyr Val Asp Ser Leu Phe Phe Leu
1               5               10


<210> 9
<211> 9
<212> PRT
<213> Artificial Sequence

<220>
<223> Synthetically prepared peptide sequence

<400> 9
Ser Leu Leu Met Trp Ile Thr Gln Cys
1               5


<210> 10
<211> 21
<212> DNA
```

```
<213> Artificial Sequence

<220>
<223> Synthetically prepared nucleic acid sequence

<400> 10
tccatgacgt tccctgacgt t                                        21

<210> 11
<211> 10
<212> PRT
<213> Artificial Sequence

<220>
<223> Synthetically prepared peptide sequence

<400> 11
Lys Ala Val Tyr Asn Ala Phe Ala Thr Met
 1               5                   10
```

**Description**

This application claims priority under 35 U.S.C. § 119(e) to U.S. Provisional Application No. 60/640,727, filed on December 29, 2004, entitled METHODS TO TRIGGER, MAINTAIN AND MANIPULATE IMMUNE RESPONSES BY TARGETED ADMINISTRATION OF BIOLOGICAL RESPONSE MODIFIERS INTO LYMPHOID ORGANS; the disclosure of which is incorporated herein by reference in its entirety.

BACKGROUND OF THE INVENTION

Field of the Invention

Embodiments described herein relate to methods to improve the efficacy and therapeutic index of a broad range of biological response modifiers that function as potentiators or modulators of immune responses mediated by B cells, CD4$^+$ T cells, CD8$^+$ T cells (including helper, regulatory, and / or cytotoxic T lymphocytes), as well as NK and NKT cells, by administering such biological response modifiers into secondary lymphoid organs such as lymph nodes.

Description of the Related Art

Immune responses can include the generation of antibodies, T helper (Th) cells and cytotoxic T lymphocytes (CTLs). Administration of biological response modifiers (BRMs) offers the possibility of manipulating immune responses since the co-stimulatory environment can decisively influence the quality and magnitude of immune responses. There are three classes of such molecules:
1. Cytokines, chemokines and co-stimulating molecules.
2. Molecules that trigger cytokine, chemokine formation and modulate the expression of co-stimulating molecules.
3. Antagonists or agonists for cytokine, chemokines and co-stimulating molecules or their receptors, for example, including antibodies, siRNA and antisense oligos.
Current immunization protocols typically deliver antigens and BRMs to non-lymphoid areas. Examples of such delivery include intravenous (i.v.), intramuscular (i.m.), subcutaneous (s.c.), intradermal (i.d.) injection, and the like. Also, such non-lymphatic delivery generally requires that the BRMs are delivered at high concentrations or in large quantities. One problem with the non-lymphatic delivery of BRMs is that there are often severe adverse effects (including adverse clinical events). The undesirable side effects associated with non-lymphatic administration of many potent BRMs include toxicity and inflammation, and are due to excessive stimulation of signal transduction pathways that, although resulting in immune modulation, have collateral effects. In the short term such activation of the immune system may lead to flu-like symptoms including fever, muscle pain, joint pain, fatigue, malaise, and/or nausea and the like. Over the long term more serious symptoms may develop. For example, both, interferon-alpha and interferon-beta have been associated with autoimmune phenomena (Weber RW, Curr Opin Allergy Clin Immunol. 4:277-83, 2004 (incorporated herein by reference in its entirety)). Also, repeated administration of vaccine adjuvants such as CpG has been found to cause lymphoid follicle destruction and immunosuppression (Heikenwalder M, et al., Nat Med. 10(2):187-92, 2004 (incorporated herein by reference in its entirety)).
The toxicity of treatment with co-stimulatory cytokines, exemplified by the major T cell growth hormone IL-2 is associated with severe toxicity resembling the clinical manifestations of septic shock. A study by Atkins et al., analyzed the side-effects of such treatment in 270 patients. (Atkins, MB, et al., Journal of Clinical Oncology, 17(7): 2105, 1999 (incorporated herein by reference in its entirety)). In the study, hypotension occurred in the majority (64%) of patients. Other observed symptoms included supraventricular tachycardia (17% of patients), adult respiratory distress syndrome (4% of patients), respiratory failure, coma (1% of patients), elevations of creatinine levels, and elevated bilirubin levels. Moreover, nausea, vomiting, and mental status changes were common among the observed patients. Infections occurred frequently (15% of patients), and life threatening sepsis occured in 3% of the patients. In addition, 2.2% of the 270 patients observed in the study died from treatment-related toxicity. Severe toxicity was also observed for another co-stimulatory cytokine, IL-12, in a phase I study where fever/chills, fatigue, nausea, vomiting, headache, anemia, neutropenia, lymphopenia, hyperglycemia, thrombocytopenia, and hypoalbuminemia were observed. In a phase II study, of the 17 patients receiving IL-12 in the phase II study, 12 patients were hospitalized and 2 patients died. (Reviewed in M. Colombo and G. Trinchieri, Cytokine & Growth Factor Reviews, 13(2):155-168, 2002 (incorporated herein by reference in its entirety)). Thus, many potentially useful BRMs cannot be effectively used because of the severe side effects, particularly when delivered non-lymphatically (e.g., intravenously, intramuscularly, subcutaneously or intradermally).
For example, signal transduction initiated by toll-like receptors (TLRs), such as TLR9 - a natural receptor for unmethylated CpG motifs - results in activation of transcription factors including NF-kB, with substantial impact on the innate and adaptive immunity. However, practical application of new adjuvants such as CpG oligodeoxynuclotides (CpG ODNs) remains a challenge since prominent systemic activation of NF-kB may result in severe side effects reminiscent of septic shock, thus limiting their therapeutic index (TI) and practical effectiveness. Similarly, non-lymphatic use of other ligands for TLRs such as double stranded RNAs (dsRNAs) can result in activation of pathways resulting in generation of TNF-alpha, thus mimicking a toxic/septic syndrome and precluding safe and effective clinical use of such reagents. Indeed, although the immunopotentiating effects of dsRNA have long been known from *in vitro* studies, attempts to us it as an adjuvant *in vivo* have repeatedly failed due to its toxic effects. Comparably based concern over toxicity has limited the clinical use of imiquimod and resiquimod to topical applications.

As another example, IL-2, IL-12, and interferons, despite the potential benefit of their potent effects on T cell proliferation and innate immunity, are extremely toxic when delivered non-lymphatically. Further, antibodies that are strong modulators of T cell response and blockers of T cell tolerance, such as anti-CTLA4, are quite toxic and result in autoimmune syndromes when delivered non-lymphatically. Finally, antibodies that block the generation of T1 cells (such as anti-IFN-gamma, anti-IL-12) and cytokines that promote the generation of T regulatory responses (such as TGF-beta, IL-10, IL-4), have potential toxic effects (e.g., impairing immune responses), and/or limited efficacy due to pharmacokinetics when delivered non-lymphatically. In fact, due to considerations primarily related to the TI, for 70 years only one adjuvant, alum, was approved for large scale vaccinations despite substantial progress in the area of immune manipulation.

Methods to improve on the safety and efficacy of BRM use are the desired and described below.

Summary of the Invention

Some embodiments relate to methods of improving the therapeutic index of a BRM. The methods can include, for example, the steps of administering a BRM to a secondary lymphoid organ of a subject in a lymphatically effective dose wherein the lymphatically effective dose avoids or reduces a BRM-related adverse clinical event as compared to use of a non-lymphatically effective dose of the BRM. The modulated immune response can be antigen-specific. The antigen can be endogenously present in the secondary lymphoid organ of the subject. The method further can include detecting little or no systemic toxicity or inflammation, for example, by detecting the presence or absence of toxicity or inflammation. Preferably, a lymphatically effective dose is at least 10-fold less than a non-lymphatically effective dose. The administering step can include co-administration of the antigen with the BRM. The administering step can include administering the antigen proximal in time to the BRM. The administering step can include administering a lymphatically effective dose of a BRM directly to a secondary lymphoid organ of the subject. The administering step can include administering the antigen to an identified area of relatively high lymphatic drainage that drains to a secondary lymphoid organ of the subject. The administering step can include administration directly to a lymph node or lymph vessel. The modulation of the immune response can include an increased or a decreased response to the antigen. The modulation of the immune response can include a shift toward a humoral response to the antigen, a shift in relative proportions of individual antibody isotypes of antigen-specific antibodies, a shift toward a cellular response to the antigen, a shift toward a T1 response to the antigen, a shift toward a T2 to the antigen, a shift toward a CTL response to the antigen, a shift toward a T helper cell response to the antigen, a shift toward a T regulatory cell response to the antigen, or any other modulation. The BRM can include a cytokine, for example, IL-12, IL-18, GM-CSF, flt3-ligand, interferons, TNF-alpha, and the like. The BRM can include a chemokine, for example, IL-8, MIP-3alpha, MIP-1alpha, MCP-1, MCP-3, RANTES, and the like. The BRM can include a toll-like receptor ligand, for example, dsRNA, or a DNA comprising a CpG sequence. The DNA can be, for example, an oligonucleotide, a plasmid, or the like. The toll-like receptor ligand can be, for example, peptidoglycan, LPS, LPS analogues, flagellin, lipoteichoic acid, imiquimode, resiquimod, microbial nucleic acids, CpG-containing oligonucleotides, ds RNA, polyI:C, and the like. The BRM can also comprise a small molecule, antibody, or engineered soluble ligand that acts as an agonist or an antagonist specific for a target selected from the group consisting of cellular receptors, co-stimulatory receptors, cytokine receptors, chemokine receptors, signal transduction elements, and transcriptional regulators. The BRM can include an antibody specific for a co-stimulatory receptor, for example, anti-CD40, anti-CTLA4, anti-CD3, anti-CD28, and anti-OX40; or other agonists of these receptor molecules. Similarly the BRM can be an antagonist of an inhibitory receptor, for example an anti-CD25 antibody. Further, the BRM can be a molecule that increases the activity of T regulatory cells, such as TGF-β, IL-10, IL-4 antibodies or antagonists for pro-inflammatory cytokines. Preferably, the antigen is not the BRM. The antigen can include a tumor-associated antigen, for example. Also, the antigen can include a microbial antigen, for example, one associated with a protist, fungi, bacterium, virus, prion, or the like. The antigen can also include an allergen, a toxin or toxoid, a disease-matched antigen, and the like.

Other embodiments relate to methods of modulating an antigen-specific immune response, the method comprising the step of administering a BRM to a secondary lymphoid organ of a subject in a dose sufficient to obtain a modulated immune response.

Other embodiments relate to methods of modulating an immune response to an antigen that include, for example, co-administering a BRM and said antigen to a secondary lymphatic organ whereby the antigen-specific immune response is modulated, wherein the modulation does not consist essentially of an augmented CTL response.

Still further embodiments relate to methods of generating an immune response while minimizing adverse side effects of a biological response modifier, which methods can include, for example, administering a BRM to a secondary lymphoid organ, wherein the BRM can include a toll-like receptor ligand.

Some embodiments can alternatively include observing less severe side effects than would result from a non-lymphatically effective dose.

In some aspects, a dose of BRM is delivered that is effective at triggering, maintaining or manipulating an immune response, while having little or no systemic or local toxicity.

Some embodiments relate to use of a BRM in the manufacture of a medicament suitable for administration to a secondary lymphoid organ of a subject.

Some embodiments relate to use of a BRM in the manufacture of a medicament suitable for modulating an immune response in a subject without causing a BRM-related adverse clinical event.

Some embodiments relate to use of a BRM in the manufacture of a medicament that increases the therapeutic index of the BRM and that is suitable for modulating an immune response in a subject.

Some embodiments relate to use of a BRM and an antigen in the manufacture of a medicament for modulating an immune response in a subject that is specific to said antigen and that is suitable for injection into a secondary lymphoid organ of a subj ect.

Other embodiments relate to a composition comprising a lymphatically effective dose of a BRM, wherein said lymphatically effective dose comprises an amount of a BRM that is relatively nontoxic and wherein said lymphatically effective dose is less than a non-lyphatically effective dose.

Other embodiments relate to a composition comprising a lymphatically effective dose of a BRM, wherein said lymphatically effective dose comprises an amount of a BRM that is relatively non-toxic and wherein the amount of the BRM is insufficient to be a non-lyphatically effective dose.

Other embodiments relate to a composition comprising a lymphatically effective dose of a BRM and an antigen, wherein said lymphatically effective dose comprises an amount of a BRM that is relatively non-toxic and wherein the amount of the BRM is insufficient to be a non-lyphatically effective dose. In preferred embodiments the lymphatically effective dose of a BRM used in such a composition is at least ten-fold less than the corresponding non-lyphatically effective dose.

Brief Description of the Drawings

Figure 1 shows that the induction of acute phase reaction (A) and increased spleen cellularity as an indication of splenomegaly (B) occurred at a dose of 10nM of CpG ODN irrespective of the tested route, but did not occur at lower doses.

Figure 2 presents the effect of titrated CpG ODN treatment on splenocyte subset composition.

Figure 3 shows the effect of CpG ODN treatment on *in vivo* maturation of dendritic cells measured as CD80 and CD86 expression.

Figure 4 shows the induction of cytotoxic responses against a model antigen (OVA) by co-administration of antigen and CpG ODN at doses devoid of systemic acute phase reaction.

Figure 5 shows the induction of immunity to a cancer antigen by intralymph node co-administration of a selected peptide epitope with two different BRMs.

Figure 6 shows the induction of functional immunity resulting in the clearance of human tumor cells within lung parenchyma, by intra lymph node co-administration of a selected peptide epitope with two different BRMs.

Figure 7 shows decreased immune responsiveness to the self PSMA 288-297 epitope as compared to the non-self PSMA 730-739 epitope by subcutaneous immunization in combination with IFA.

Figures 8A-8E show induction of cytotoxic response by co-administration of various peptides with adjuvant (polyI:C) into lymph nodes using self and non-self epitopes.

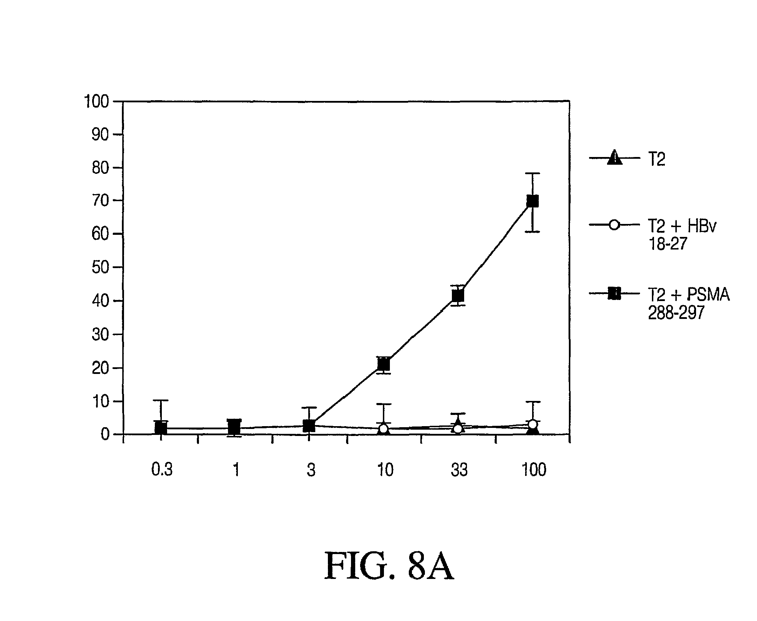

Figure 8A shows lysis induced by immunization with PSMA 288-297 on T2 cells pulsed with the peptide.

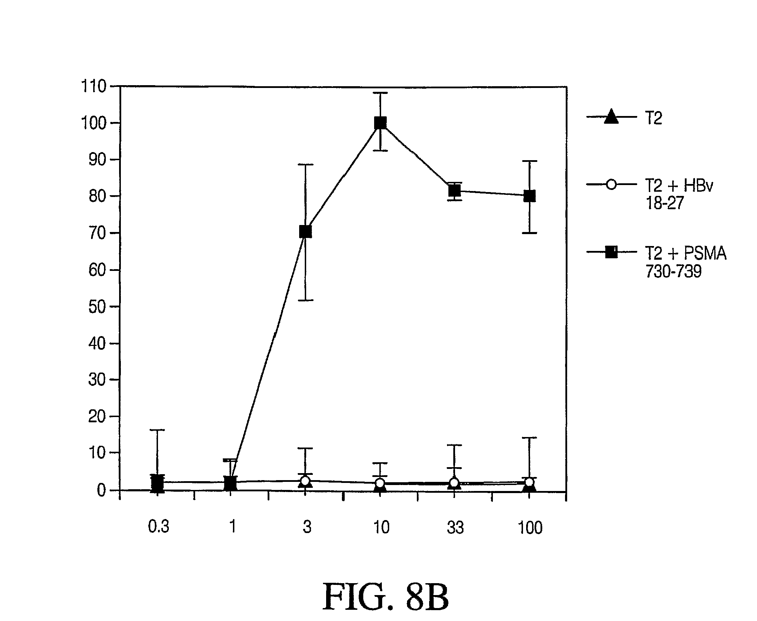

Figure 8B shows lysis induced by immunization with PSMA 730-739 on T2 cells pulsed with the peptide.

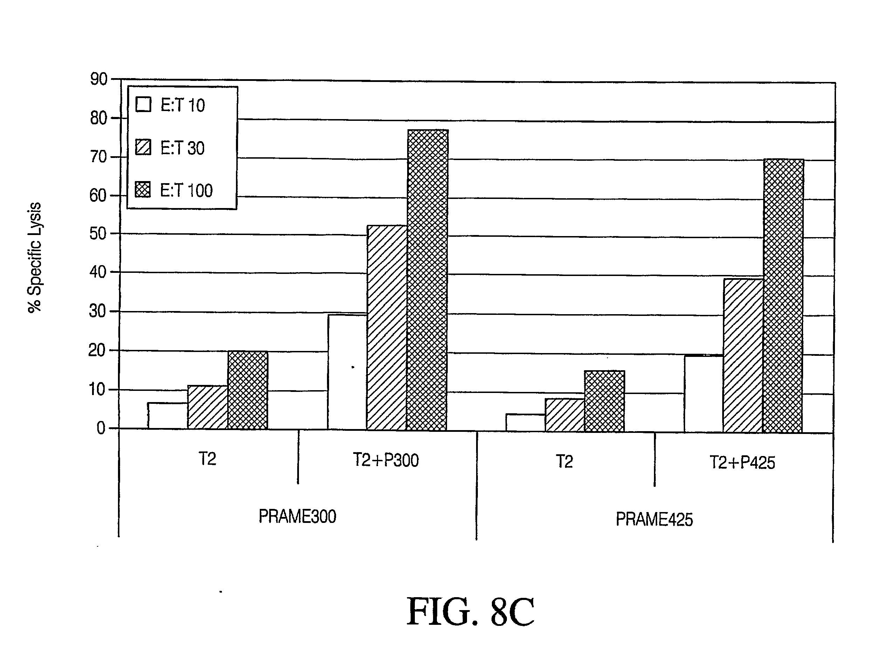

Figure 8C shows lysis induced by immunization with PRAME 425-433 and PRAME 300-309 on T2 cells pulsed with the peptide.

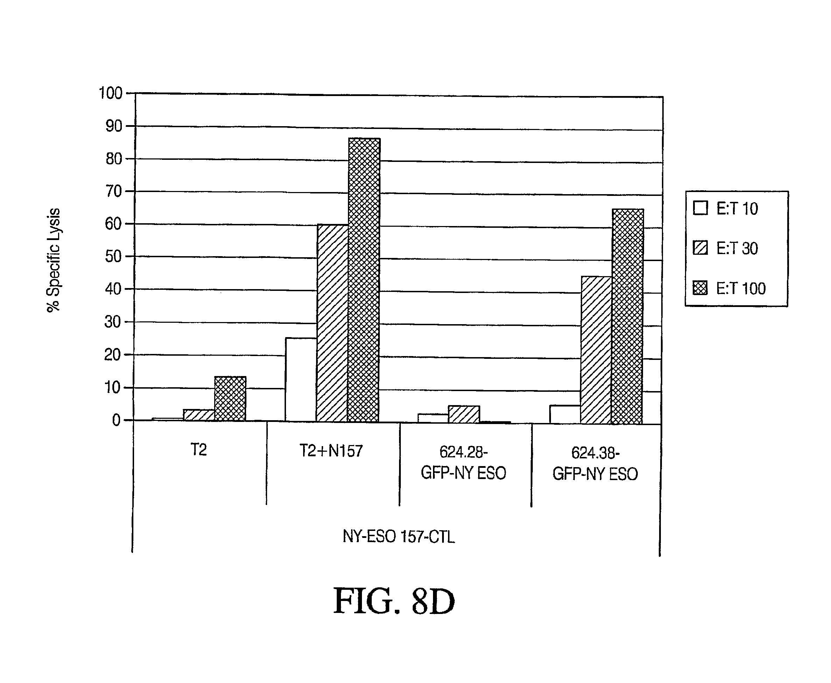

Figure 8D shows lysis induced by immunization with NY-ESO-1 157-165 on T2 cells pulsed with the peptide on the left and on 624 cells transformed to express a GFP/NY-ESO-1 fusion protein on the right.

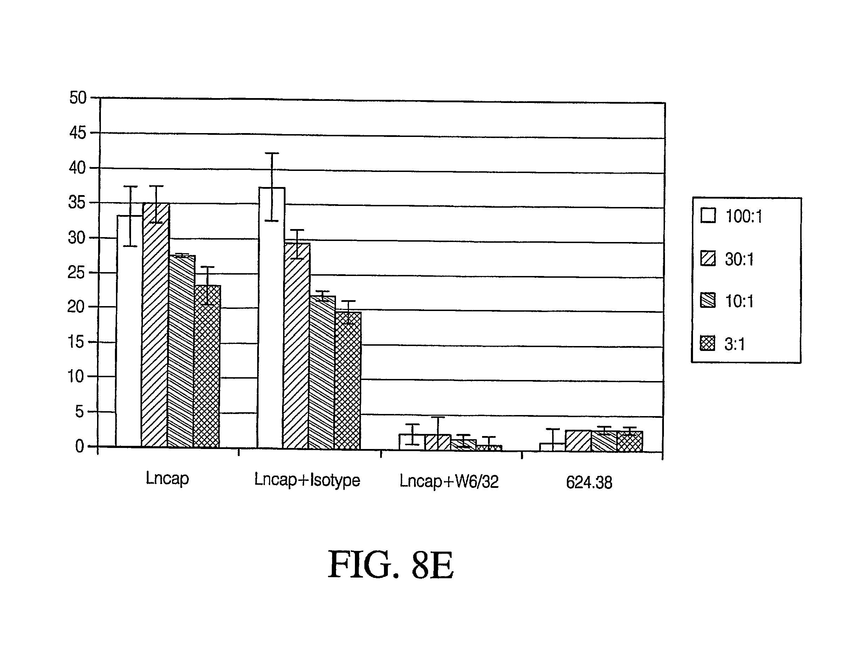

Figure 8E shows lysis induced by immunization with PSMA 288-297 on a PSMA-expressing human tumor cell line (LNCap).

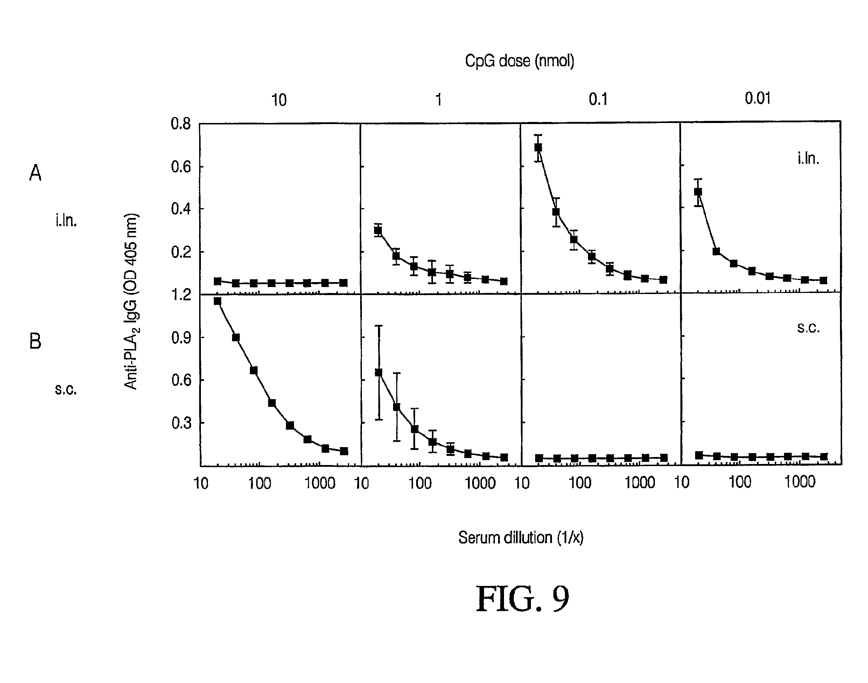

Figure 9 shows the induction of antibody response against PLA2 by co-administration of antigen and CpG ODN at doses devoid of systemic acute phase reaction.

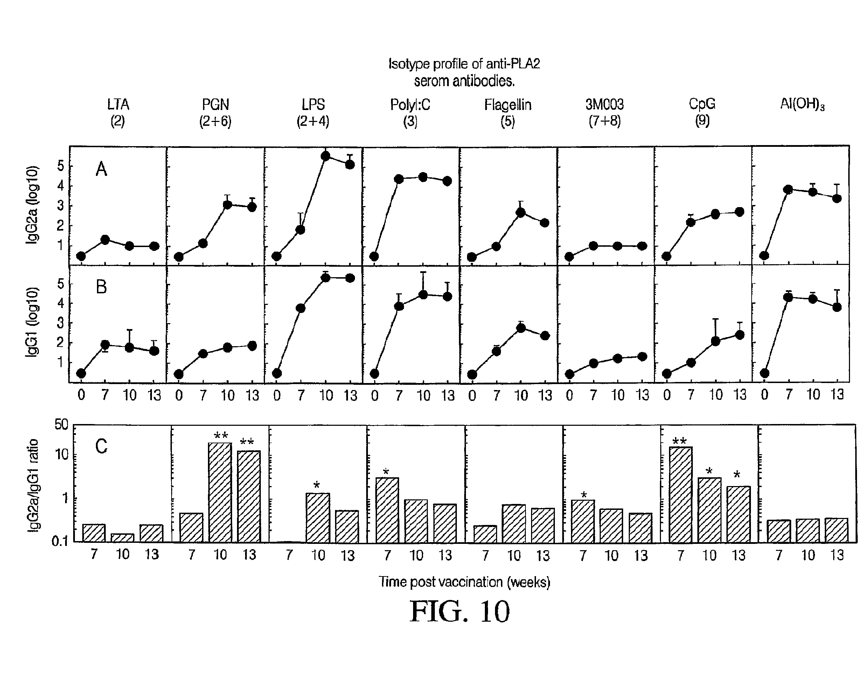

Figure 10 shows an isotype profile of anti-PLA2 serum antibodies in CBA/J mice vaccinated on day 1, 15 and 29 with 0.1 μg PLA2 and the indicated Toll-like receptor ligands or the reference adjuvant A1(OH)3.

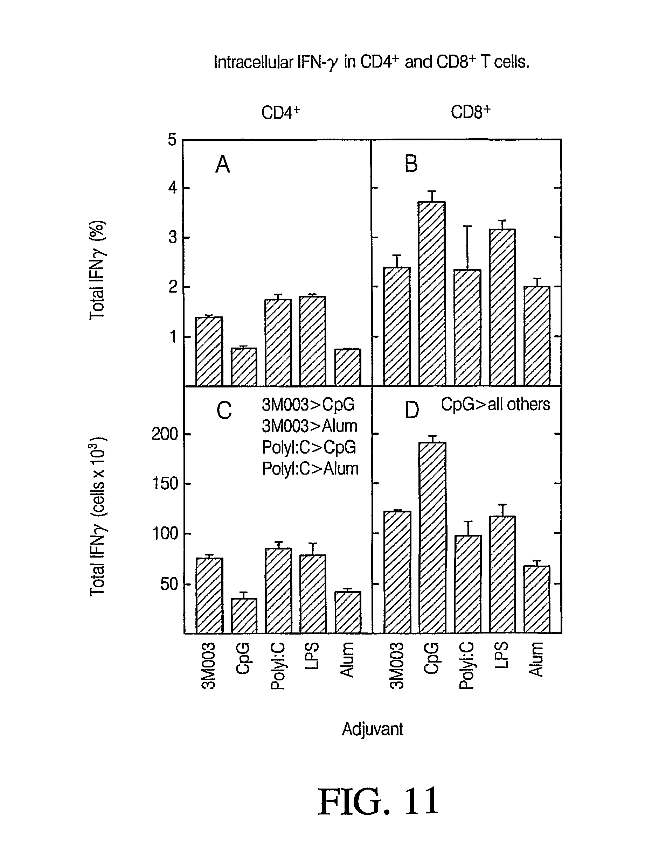

Figure 11 shows intracellular IFN-γ in the CD4+ and CD8+ T cells of mice injected with phospholipase A2 and various TLR ligands.

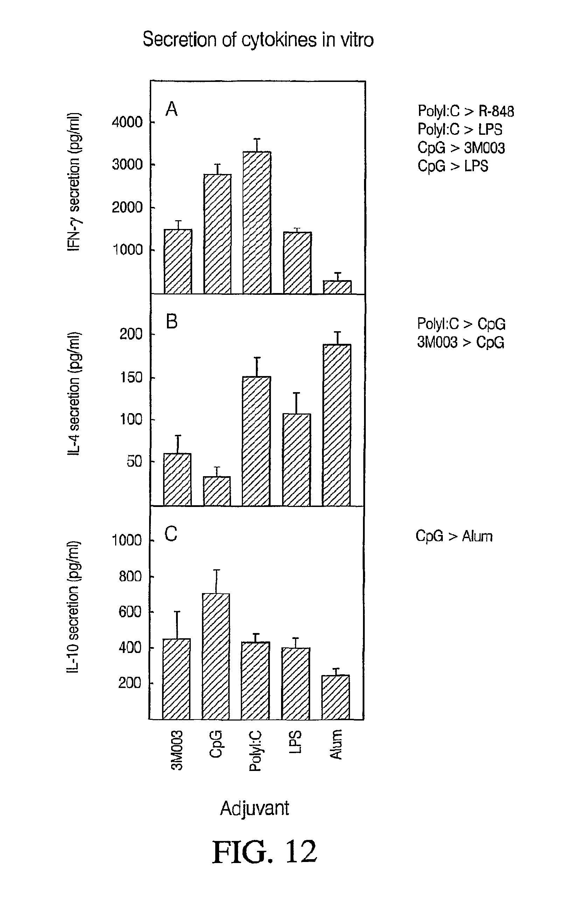

Figure 12 shows the levels of secretion of cytokines in vitro (in mice injected with phospholipase A2 and various TLR ligands).

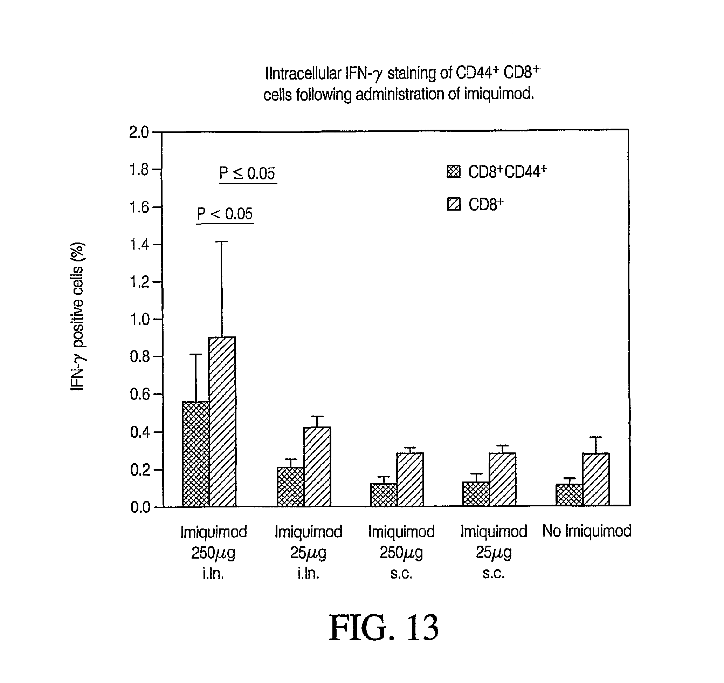

Figure 13 shows the percent of CD8+ and CD44+ cells positive for intracellular IFN-γ following in vitro restimulation with antigen (following intranodal or subcutaneous administration of imiquimod during immunization).

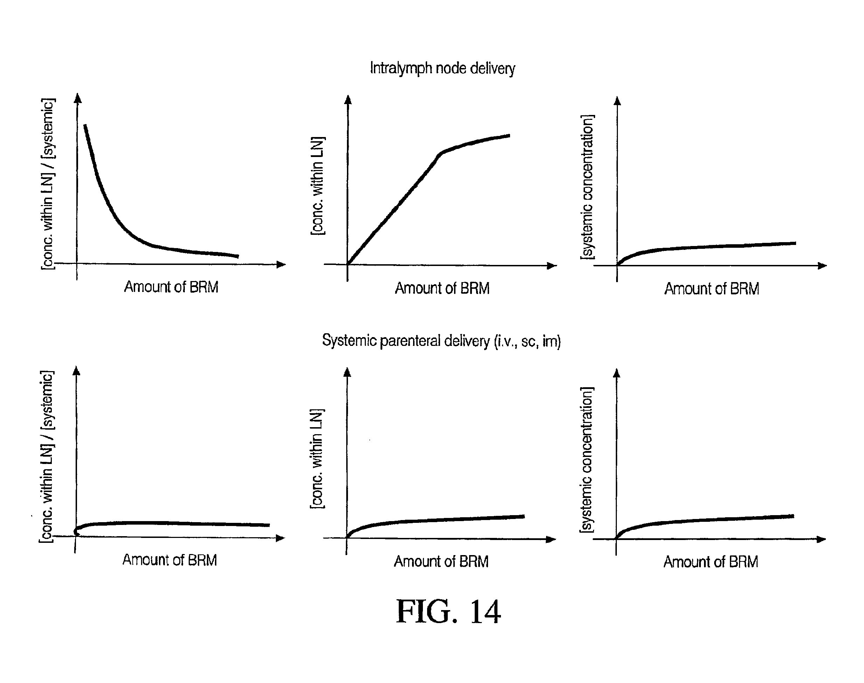

Figure 14 depicts a model explaining the efficacy and improved TI by co-administration of antigen and BRMs into lymph nodes.

Detailed description of the preferred embodiments

Definitions

Non-lymphatic administration — In preferred embodiments, non-lymphatic administration means delivery in a manner such that the effects of the delivered substance are seen throughout the body. For example, this can include intravenous administration, as well as administration subcutaneously, intradermally, intramuscularly, orally, or in any other manner so that the agent can be absorbed into the tissues or fluids of the body generally. In other embodiments, non-lymphatic administration can mean delivery in a manner such that the effects of the delivered substance are seen in substantial portions, regions, tissues, systems, or the like, of the body, even if not seen in all parts thereof.

Intralymphatic administration — In preferred embodiments, intralymphatic administration means delivery in a manner such that effects are focused generally in the lymphatic system; that is, the agent is preferentially absorbed into one or more organs of the lymphatic system. In various embodiments, intralymphatic administration can include administration(s) directly into a primary or secondary lymphoid organ, or into a lymphatic vessel or to a site of relatively high lymphatic drainage. In preferred embodiments delivery is into a secondary lymphoid organ or into a vessel or to a site of relatively high lymphatic drainage. In some embodiments, administering a BRM to a secondary lymphoid organ of a subject can be done directly by administering a BRM directly to the secondary lymphoid organ or indirectly by administering the BRM to an identified area of relatively high lymphatic drainage.

Therapeutic Index (TI) — In some embodiments this can be defined quantitatively as the ratio of $LD_{50}$ to $ED_{50}$. $ED_{50}$ is the dose of a drug that is pharmacologically effective for 50% of the population exposed to the drug or a 50% response in a biological system that is exposed to the drug. $LD_{50}$ is the chemical dose lethal to 50 percent of a test population. In other preferred embodiments the term also can be defined qualitatively to indicate the difference between the dose at which an agent becomes medically useful and the dose at which undesirable effects (adverse clinical events) become apparent, or impair or eliminate usefulness, without reference to any particular quantitative measurement.

Secondary lymphoid organs — organs where lymphocytes reside and encounter antigen. Examples include lymph nodes, adenoids and tonsils, the appendix, the spleen, and peyer's patches of the gut. Generally, primary lymphoid organs are those in which lymphocytes develop (thymus, bursa, bone marrow).

Non-lymphatically effective dose - The amount of a BRM or antigen effective to modulate an immune response to an antigen in order to obtain a desired outcome when using non-lymphatic administration. In alternative embodiments the immune response is characterized without reference to antigen specificity.

Lymphatically effective dose — The amount of a BRM or antigen effective to modulate an immune response to obtain a desired outcome when using intralymphatic administration. In some embodiments, the immune response is characterized without reference to antigen specificity. In other embodiments, the immune response is antigen-specific. Generally a lymphatically effective dose can be small enough to reduce or avoid adverse clinical events that can occur from use of a non-lymphatically effective dose.

Modulate an immune response — Any change in an immune response. The change can involve an increase, decrease, shortening, prolongation, shift in time of appearance, peak, or disappearance, of any parameter of the response, or a change in the balance of components of the response that can result in a desired outcome. The response can comprise B cells, CD4+ T cells, CD8+ T cells (including helper, regulatory, and / or cytotoxic T lymphocytes), NK cells, and NKT cells, and any of the soluble molecules they produce in response to antigenic stimulation. In preferred embodiments the change in an immune response is specific to one or more specific antigens.

BRM-related adverse clinical event — Any systemic or localized side-effect arising from administration of a BRM that is undesired including without limitation acute phase reaction, flu-like symptoms, fever, chills, muscle pain, joint pain, fatigue, malaise, nausea, vomiting, headache, anemia, neutropenia, lymphopenia, hyperglycemia, thrombocytopenia, hypoalbuminemia, systemic immune activation, splenomegaly, lymphoadenopathy, toxicity, septic shock, hypotension, supraventricular tachycardia, adult respiratory distress syndrome, mental status changes, elevated bilirubin levels, elevated creatinine levels, and the like.

Desired outcome — Any positive effect on the disease or condition being treated. In some embodiments, the positive effect comprises a decrease in the severity, frequency, or duration of one or more symptoms caused by the disease or condition being treated. In other embodiments, the positive effect comprises a decrease in the mortality of the disease or condition being treated. In some embodiments, the positive effect comprises remission of the disease or condition being treated. In yet other embodiments, the positive effect comprises elimination of the disease or condition being treated. In still other embodiments the desired outcome comprises a detectable increase, decrease, shift, or other modulation of an immune response as compared to what is, could be, or would be expected to be, obtained without use of a BRM.

Embodiments described herein relate to methods to trigger, maintain, and manipulate immune responses by targeted administration of biological response modifiers (BRMs) to lymphoid organs. Some embodiments relate to methods to improve the efficacy and therapeutic index (TI) of a broad range of BRMs by administering the BRMs into secondary lymphoid organs, such as lymph nodes. The BRMs can act as potentiators or modulators of specific immune responses mediated, for example, by B cells, T helper (Th) cells and / or cytotoxic T lymphocyte (CTL) cells. Many BRMs are underutilized or not utilized at all in many contexts because of their adverse side effects. Further embodiments relate to methods of utilizing such BRMs while avoiding some or all of their adverse side effects.

Some embodiments relate to methods that are based upon the unexpected observation that intralymphatic administration of a BRM results in an improved therapeutic index for the BRM, such that immunological effectiveness can be obtained while using doses small enough to avoid clinically relevant toxicity. It was not clear *a prior* whether administration to secondary lymphoid organs (where the interaction between various cell populations important for the generation of immune responses is optimal) of BRMs, as described herein, with various types of antigens would result in improvement of TI or efficacy of vaccination or active immunotherapy, since the normal sequence of antigen exposure and subsequent interaction between antigen presenting cells (APCs), T and B cells is different. Thus, it was previously unknown whether inadvertent stimulation or over-stimulation of immune cells by BRMs might have resulted in decreased responses or toxicity. As described herein it has been discovered that this is not the case. Some embodiments relate to a methodology to address these issues and take full advantage of classes of existing or potential BRMs that although potent, pose safety considerations when delivered non-lymphatically.

In some preferred embodiments, the methods can include the administration of BRMs to secondary lymphoid organs, or administration into the lymphatic vessels draining into such secondary organs. In the lymphoid organs, antigen presenting cells (APCs), innate immune cells, B cells, Th cells and CTLs are in geographic proximity, for example, within the same microenvironment. Thus, the predominant locus for generation of immune responses is within secondary lymphoid organs where the proximity between these cells is optimal. Some embodiments relate to methods of delivering BRM at dose that triggers, maintains, manipulates or otherwise modulates an immune response, and which also avoids localized toxicity.

In some embodiments the methods can include co-administration of antigen with the BRM. However, in other embodiments the methods relate to modulating the immune response to antigen that is already present, but unrecognized or only recognized in a suboptimal fashion by the immune response within the lymphoid environment. One example is metastatic disease in draining lymph nodes of a tumoral process. In such embodiments the antigen preferably is not co-administered, while in some embodiments, it can be co-administered.

In some embodiments the immune response is characterized without reference to a specific antigen. Instead the modulation is of the general character of an immune response such as a shift toward or away from a T1 or T2 response, or changing the absolute or relative amount of an antibody isotype or subset of T cells, or stimulating or inhibiting the proliferation or activity of any of the cell types of the immune system (including, for example, B cells, T cells, NK cell, NKT cells, and antigen presenting cells and the like).

Administration into the secondary lymphoid organs can result in increased TI by increasing the concentration of BRM in the microenvironment where the antigen is processed and presented to immune cells, while minimizing non-lymphatic exposure to BRMs. Both antibody and T cell responses (such as Th cells and CTLs) can be increased or optimized by this methodology, without undesired non-lymphatic exposure to problematic amounts of BRMs. In fact, the overall efficacy of combinations of antigen and BRM can be substantially increased by this methodology. In general, such methods can be used to trigger, amplify, suppress, or restore immune responses against microbial and tumor-associated antigens, in prophylactic or therapeutic fashion.

Potential target microbes include, without limitation, hepatitis viruses (e.g., C, B and delta), herpes viruses, HIV, HTLV, HPV, EBV, and the like. Potential target tumor antigens include, without limitation, cancer-testes antigens (e.g., NYESO-1, PRAME, SSX2, MAGE, LAGE, GAGE, etc.), tissue specific antigens (e.g., Melan-A, tyrosinase, gp100, PSMA, etc.) and oncofetal antigens (e.g., CEA, etc.), associated with carcinomas, sarcomas and other types of tumors, and the like. Numerous exemplary epitopes, epitope analogs, and combinations or antigens from tumor antigens and methods of using the same are found in U.S.'Application Nos. 10/117,937, filed on April 4, 2002, 10/657,022, filed on Septebmer 5, 2003, 11/067,159 (filed on February 25, 2005, Pub. No. 20050221440), 11/067,064 (filed on February 25, 2005, Pub. No. 20050142144) all entitled EPITOPE SEQUENCES; Application No. 10/871,708 and Provisional Application No. 60/580,969, both filed on June 17, 2004 and entitled COMBINATIONS OF TUMOR ASSOCIATED ANTIGENS IN COMPOSITIONS FOR VARIOUS TYPES OF CANCERS; and U.S. Provisional Application Nos. 60/581,001 and 60/580,962, both filed on June 17, 2004, and respectively entitled SSX-2 ANALOGS and NY-ESO PEPTIDE ANALOGS, each of which is incorporated herein by reference in its entirety. Vectors or formulations to deliver antigens can include peptides, recombinant proteins, viruses or bacteria, recombinant nucleic acids or cells encompassing the above listed agents, and the like.

Exemplary BRMs can include, without limitation, cytokines such as IL-12, IL-18, GM-CSF, flt3 ligand (flt3L), interferons, TNF-alpha, and the like; and chemokines such as IL-8, MIP-3alpha, MIP-1alpha, MCP-1, MCP-3, RANTES, and the like. In addition, BRMs can be, without limitation, molecules that trigger cytokine or chemokine production, such as ligands for TLRs (peptidoglycans, LPS or analogues, CpG ODNs, dsRNAs, small molecules that bind to TLRs such as imiquimode, and the like). Antibodies, small organic molecules, and engineered soluble ligands that bind to co-stimulatory molecules (anti-CD40, CTLA-4, anti-OX40, and the like), cellular receptors, cytokine receptors, chemokine receptors, signal transduction elements, or transcriptional regulators can be used as BRMs in the context described herein. Finally, BRMs can include small interfering RNA (siRNA) that alter expression of proteins which in turn are regulators of the immune system.

Further, the disclosed methods can, for example, be used to modulate or suppress undesired immune responses associated with inflammatory, allergic or autoimmune disorders, by enabling immune deviation, generation of T regulatory cells or immune tolerance. For example, one or more BRMs which suppress certain immune responses or which modulate responses in a manner that is favourable for the above-mentioned disorders can be administered alone or in combination with an antigen to a secondary lymphoid organ. The one or more BRMs can be delivered in a low amount, which can minimize or avoid the adverse effects normally associated with the one or more BRMs. For T1-mediated autoimmune diseases, antigens can include epitopes derived from insulin, GAD65, myelin basic protein, proteolipid protein, MOG, collagen II, heat shock proteins, etc. BRMs may be IL-4, TGF-beta, IL-10, and the like; or molecules that trigger their production. In addition, BRMs may be antibodies that enable the mechanisms listed above. Examples include anti-IFN-gamma, anti-IL-12, and including their use in combination with the BRMs listed above to amplify the overall effect. For T2 mediated diseases, the BRMs that can be used include T1-activating antigens, including those listed in the previous section. Together, such methods can be applied prophylactically or therapeutically in diseases such as autoimmune diabetes, multiple sclerosis, rheumatoid arthritis and various allergies including asthmatic disorder with allergic aetiology.

As used herein BRM can refer to any molecule that modulates the activity of the immune system, or the cells thereof, through an interaction other than with an antigen receptor. BRM is also commonly applied to complex biological preparations comprising such molecules in which the active entity, or entities, without regard for whether the active component(s) of the mixture had been defined. Examples of complex biological preparations used as BRMs include OK-432, PSK, AIL and lentinan. In preferred embodiments of the invention the active component(s) of such a mixture are defined. In other preferred embodiments of the invention BRMs sourced from complex biological preparations are at least partially purified, or substantially purified, for example OK-PSA (Okamoto et al., Journal of the National Cancer Institute, 95:316-326, 2003 which is incorporated herein by reference in its entirety) or AILb-A (Okamoto et al., Clinical and Diagnostic Laboratory Immunology, 11:483-495, 2004 which is incorporated herein by reference in its entirety). In particularly preferred embodiments the BRM is of defined molecular composition. BRMs include immunopotentiating adjuvants that activate pAPC or T cells including, for example: TLR ligands, endocytic-Pattern Recognition Receptor (PRR) ligands, quillaja saponins, tucaresol, cytokines, and the like. Some preferred adjuvants are disclosed in Marciani, D.J. Drug Discovery Today 8:934-943, 2003, which is incorporated herein by reference in its entirety. In one review, it was reported that a BRM was injected into a lymph node (Sadao et al., "Locoregional immunotheraphy-Topics at the 13th and 14th Meeting of the Japanese Research Society for Surgical Cancer Immunology," Biotherapy 9(7):845-851 (1995) (incorporated herein by reference in its entirety)). However, the report provided minimal detail and no guidance regarding the exact response caused by the injection. The report did not disclose any antigen specific immune response, for example, a B cell or T cell response due to the injection.

One class of BRM includes mostly small organic natural or synthetic molecules, which exert immune modulating effects by stimulating pathways of innate immunity. It has been shown that macrophages, dendritic and other cells carry so-called Toll-like receptor (TLRs), which recognize pathogen-associated molecular patterns (PAMPs) on micro-organisms (Thoma-Uszynski, S. et al., "Induction of direct antimicrobial activity through mammalian toll-like receptors," Science 2001. 291:1544-1547; Akira, S., "Mammalian Toll-like receptors," Curr Opin Immunol 2003. 15: 5-11; each of which is incorporated herein by reference in its entirety). Ligation of the TLRs activates NF-κB and leads to the production of several important mediators of innate immunity, e.g., IFN-α, IFN-γ, TNF-α, IL-1, IL-6, IL-12 and IL-18, and induces the expression of co-stimulatory molecules, i.e., B7.1 (CD80), B7.2 (CD86), and CD40, on antigen-presenting cells. It is the presence of these molecules along with presentation of microbial antigens that activates the CD4 T cells required to initiate most adaptive immune responses.

So far, eleven mammalian TLRs have been described together with a multitude of natural and synthetic ligands, e.g., lipopolysaccharide, various glycans and mannans, the mycobacterial extract muramyl dipeptide, bacterial flagellin, bacterial dsDNA (which contains CpG motifs), viral and synthetic dsRNA (e.g., polyI:C) and viral ssRNA (Ahmad-Nejad, P. et al., Eur J Immunol 2002. 32: 1958-1968 ; Chamaillard, M., et al., Nat Immunol 2003. 4: 702-707; McSorley, S. J.,

et al., J Immunol 2002. 169: 3914-3919; Krieg, A. M.,. Annu Rev Immunol 2002. 20: 709-760; Heil, F., et al., Science 2004. 303: 1526-1529; Diebold, S. S.et al., Science 2004. 303: 1529-1531; each of which is incorporated herein by reference in its entirety). CpG especially (TLR-9 ligand) has shown widespread experimental application and clinical potential as adjuvant by allowing efficient maturation of antigen-presenting cells and subsequent activation of antigen-specific lymphocytes (Krieg, A. M., Annu Rev Immunol 2002. 20: 709-760; Weigel, B. J.et al., Clin Cancer Res 2003. 9: 3105-3114; Verthelyi, D.et al., Aids 2004. 18: 1003-1008; Storni, T. et al., J Immunol 2004. 172: 1777-1785; each of which is incorporated herein by reference in its entirety). Furthermore, a new generation of purely synthetic anti-viral imidazoquinolines, e.g., imiquimod and resiquimod, has been found to stimulate the cellular path of immunity by binding the TLRs 7 and 8 (Hemmi, H. et al., Nat Immunol 3:196-200, 2002; Dummer, R. et al., Dermatology 207:116-118, 2003; Kadowaki, N. et al. J Exp Med. 194:863-9, 2001; each of which is incorporated herein by reference in its entirety). Others suggest that these compounds are better viewed as shifting, as opposed to stimulating immune responses (Brugnolo, F. et al. J Allergy Clin Immunol 111:380-8, 2003, which is incorporated herein by reference in its entirety).

The administration of a BRM with an antigen was studied. T cell responses against protein antigens are of interest for treatment and immunization against microbes and tumors. The methods can include the co-administration of a model antigen such as OVA with CpG ODN, which was studied to determine whether their administration results in favorable immune response (CTL) at doses devoid of detectable systemic acute phase reaction. Furthermore, as described more fully herein, melan-A 26-35, is a major, well-described epitope derived from a tumor-associated antigen expressed on melanoma cells, and is a model antigen for co-administered studies with the BRMs listed above. An example of particular BRMs that are potent immune modulators, and also associated with safety concerns when delivered non-lymphatically, are unmethylated CpG oligodeoxynuclotides (CpG ODN) and synthetic dsRNA (polyI:C) that bind to TLR9 and TLR3, respectively, on APC and innate immune cells.

Since a major potential application of CpG ODNs consists in cancer immunotherapy, it was administered into lymph nodes at low doses (around 0.1 nmole) to determine whether it augments immunization with the Melan-A peptide epitope. Mice carrying a transgene that expresses a human-mouse chimeric MHC class I molecule (displaying the human A2 allotype) were immunized with peptide alone or peptide admixed with CpG ODN. The peptide used was the A27L analogue of the A2-restricted Melan-A 26-35 epitope. The analogue is cross-reactive with the natural peptide and displays decreased $K_{off}$ along with increased immunogenicity.

In addition, the methodologies described herein can be utilized in the area of allergic desensitisation and in generation of antibody responses against a broad range of microbial antigens. Such methods can include the co-administration of an antigen, such as PLA2, along with CpG ODN, which was studied to determine whether such administration results in favorable immune response (antibodies) at doses low in or lacking in detectable systemic acute phase reaction.

Additional variations will be apparent to one of skill in the art. Various embodiments can specifically include or exclude the use of particular BRMs, antigens, forms of antigen, mode of administration, etc. For example, some embodiments exclude the use of BRMs OK-432, lentinan, PSK, IL-2, TNF, and IFN-gamma, individually. Other embodiments exclude the use of all of the same or more than one of the same, for example, 2, 3, 4, or 5 of the BRMs.

Additional methodology, compositions, peptides, and peptide analogues are disclosed in U.S. Provisional Application No. 60/581,001, filed June 17, 2004, , and 11/156,253, filed on June 17, 2005, both entitled "SSX-2 PEPTIDE ANALOGS;" U.S. Provisional Application No. 60/580,962, filed June 17, 2004,, and 11/155,929, filed on June 17, 2005, both entitled NY-ESO PEPTIDE ANALOGS; U.S. Patent Application No. 09/999,186, filed November 7, 2001, entitled METHODS OF COMMERCIALIZING AN ANTIGEN; U.S. Provisional Application No. 60/640,402, filed on December 29, 2004, and U.S. Patent Application No _/_,_, (Attorney Docket No. MANNK.047A) filed on even date as the instant application, both entitled, METHODS TO ELICIT, ENHANCE AND SUSTAIN IMMUNE RESPONSES AGAINST MHC CLASS I- RESTRICTED EPITOPES, FOR PROPHYLACTIC OR THERAPEUTIC PURPOSES; and U.S. Provisional Application No. 60/640,821, filed on December 29, 2004, and U.S. Patent Application No _/_,_, (Attorney Docket No. MANNK.048A filed on even date as the instant application), both entitled METHODS TO BYPASS CD4+ CELLS IN THE INDUCTION OF AN IMMUNE RESPONSE, each of which is hereby incorporated by reference in its entirety. Beneficial epitope selection principles for immunotherapeutics are disclosed in U.S. patent application Nos. 09/560,465 (filed on April 28, 2000), 10/026,066 (filed on December 7, 2001; Pub. No. 20030215425 A1), 10/005,905 (filed on November 7, 2001), 10/895,523 (filed on July 20, 2004, Pub. No. 20050130920), and 10/896,325 (filed on July 20, 2004) all entitled EPITOPE SYNCHRONIZATION IN ANTIGEN PRESENTING CELLS; U.S. Pat. No. 6,861,234(filed on April 28, 2000) and U.S. patent application No. 10/956,401 (filed on October 1, 2004, Pub. No. 20050069982 A1) both entitled METHOD OF EPITOPE DISCOVERY; U.S. patent application Nos. 09/561,571 (filed on April 28, 2000) entitled EPITOPE CLUSTERS; 10/094,699 (filed on March 7, 2002; Pub. No. 20030046714 A1) and 11/073,347 (filed on June 30, 2005) both entitled ANTI-NEOVASCULATURE PREPARATIONS FOR CANCER; and 10/117,937 (filed on April 4, 2002; Pub. No. 20030220239 A1) and 10/657,022 (filed on September 5, 2003; Publication No. 20040180354 A1), and PCT Application No. PCT/US2003/027706 (Pub. No. WO04022709A2) all entitled EPITOPE SEQUENCES, and each of which is hereby incorporated by reference in its entirety. Aspects of the overall design of vaccine plasmids are disclosed in U.S. Patent applications 09/561,572 (filed on April 28, 2000) and 10/225,568 (filed on August 20, 2002, Pub. No. 20030138808) both entitled EXPRESSION VECTORS ENCODING EPITOPES OF TARGET-ASSOCIATED ANTIGENS and U.S. Pat. App. Nos. 10/292,413 (filed on November 7, 2002; Pub. No.20030228634 A1), 10/777,053 (filed on February 10, 2004, Pub. No. 20040132088), 10/837,217 (filed on April 30, 2004) all entitled EXPRESSION VECTORS ENCODING EPITOPES OF TARGET-ASSOCIATED ANTIGENS AND METHODS FOR THEIR DESIGN; 10/225,568 (filed on August 20, 2002; Pub No. 2003-0138808), PCT Application No. PCT/US2003/026231 (Pub. No. WO 2004/018666) and U.S. Patent No. 6,709,844 and U.S. patent application No. 10/437,830 (filed on May 13, 2003, Pub. No. 20030180949) both entitled AVOIDANCE OF UNDESIRABLE REPLICATION INTERMEDIATES IN PLASMIND PROPAGATION, each of which is hereby incorporated by reference in its entirety.

Specific antigenic combinations of particular benefit in directing an immune response against particular cancers are disclosed in U.S. Provisional Application No. 60/479,554, filed on June 17, 2003, and U.S. Patent Application No. 10/871,708, filed on June 17, 2004, PCT Patent Application No. PCT/US2004/019571, U.S. Provisional Application No. 60/640,598, filed on December 29, 2004, and U.S. Patent Application No _/_,_, (Attorney Docket No. MANNK.049A), filed on even date herewith, all entitled COMBINATIONS OF TUMOR-ASSOCIATED ANTIGENS IN VACCINES FOR VARIOUS TYPES OF, each of which is also hereby incorporated by reference in its entirety.

Intranodal administration for the generation of CTL is taught in U.S. Patent Application Nos. 09/380,534; 09/776,232 (Pub. No. 20020007173 A1) now U.S. Patent No. 6,977,074; _/_,_ (Attorney Dockey No. MANNK.001CP2C1, filed on December 19, 2005; in PCT Application No. PCTUS98/14289 (Pub. No. W09902183A2) each entitled A METHOD OF INDUCING A CTL RESPONSE and in U.S. Application No. 10/871,707, filed on June 17, 2004, entitled, METHODS TO ELICIT, ENHANCE AND SUSTAIN IMMUNE RESPONSES AGAINST MHC CLASS I-RESTRICTED EPITOPES, FOR PROPHYLACTIC OR THERAPEUTIC PURPOSES, each of which is hereby incorporated by reference in its entirety.

Intranodal administration of allergen for allergy desensitization is taught in U.S. Patent No. 6,773,695 entitled MODULATION OF ALLERGIC RESPONSE, which is hereby incorporated by reference in its entirety.

The integration of diagnostic techniques to assess and monitor immune responsiveness with methods of immunization is discussed more fully in Provisional U.S. Patent Application No. 60/580,964, filed on June 17, 2004, and U.S. Patent Application No. 11/155,928, filed on June 17, 2005, both entitled IMPROVED EFFICACY OF ACTIVE IMMUNOTHERAPY BY INTEGRATING DIAGNOSTIC WITH THERAPEUTIC METHODS, each of which is hereby incorporated by reference in its entirety.

Additional relevant disclosure is present in U.S. Patent App. Nos. 11/155,288 (filed on June 17, 2005) entitled COMBINATIONS OF TUMOR-ASSOCIATED ANTIGENS IN COMPOSITIONS FOR VARIOUS TYPES OF CANCERS, 11/156,369 and 60/691,889 (both filed on June 17, 2005) both entitled "EPITOPE ANALOGS," 60/691,579 (filed on June 17, 2005) entitled METHODS AND COMPOSITIONS TO ELICIT MULTIVALENT IMMUNE RESPONSES AGAINST DOMINANT AND SUBDOMINANT EPITOPES, EXPRESSED ON CANCER CELLS AND TUMOR STROMA, and 60/691,581 (filed on June 17, 2005) entitled MULTIVALENT ENTRAIN-AND-AMPLIFY IMMUNOTHERAPEUTICS FOR CARCINOMA.

The compositions described herein can be administered to a human patient *per se,* or in pharmaceutical compositions where they are mixed with other active ingredients, as in combination therapy, or suitable carriers or excipient(s). Techniques for formulation and administration of the compounds of the instant application may be found in "Remington's Pharmaceutical Sciences," Mack Publishing Co., Easton, PA, 18th edition, 1990.

The compositions of the present invention can be prepared in combination with an acceptable pharmaceutical carrier. Suitable carriers can contain inert ingredients that do not interact with the compound. Techniques for the preparation of pharmaceutical compositions are well known in the art and for example described in Remington's Pharmaceutical Sciences (Mack Publishing Company, Easton, Pa.,) (which is incorporated herein in its entirety). Suitable pharmaceutical carriers for intravenous and other parenteral administration include, for example, sterile water, physiological saline, bacteriostatic saline (i.e., saline containing about 0.9% mg/ml benzyl alcohol), phosphate-buffered saline, Hank's solution, or Ringer's lactate.

The term "carrier" defines a chemical compound that facilitates the delivery or incorporation of a compound into cells or tissues.

The term "diluent" defines a liquid, for pharmaceuticals generally an aqueous solution, that will dissolve and can be used to dilute a compound of interest (active agent) as well as stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound.

The term "physiologically acceptable" defines a carrier or diluent that does not abrogate the biological activity and properties of a compound (active agent) and is compatible with the functioning of a living organism to which it will be delivered.

For injection, the agents of the invention may be formulated in aqueous solutions, preferably in physiologically compatible buffers such as Hanks's solution, Ringer's solution, or physiological saline buffer. The compositions may take such forms as suspensions, solutions or emulsions in oily or aqueous vehicles, and may contain formulatory agents such as suspending, stabilizing and/or dispersing agents.

The exact formulation, route of administration and dosage for the pharmaceutical compositions of the present invention can be chosen by the individual physician in view of the patient's condition. (See *e.g.,* Fingl et al. 1975, in "The Pharmacological Basis of Therapeutics", Ch. 1 p. 1, which is incoporated herein in its entirety). Typically, the dose range of the composition administered to the patient can be from about 0.5 to 1000 mg/kg of the patient's body weight. The dosage may be a single one or a series of two or more given in the course of one or more days, as is needed by the patient. Note that for particular compounds disclosed herein, suitable human dosage can be inferred from $ED_{50}$ or $ID_{50}$ values, or other appropriate values derived from *in vitro* or *in vivo* studies, as qualified by toxicity studies and efficacy studies in animals.

Example 1

High doses of CpGs (10 nmol) administered either subcutaneously or intralymphatically induce significant acute phase response and splenomegaly.

It is well known that microbial components such as endotoxin and bacterial DNA can induce severe adverse effects, e.g., systemic immune activation, splenomegaly, lymphoadenopathy and septic shock (reviewed in Schmidt, U., H. Wagner, and T. Miethke, "CpG-DNA upregulates the major acute-phase proteins SAA and SAP," Cell Microbiol 1: 61, 1999 (which is incorporated herein in its entirety)). These adverse effects are usually preceded by a so-called acute-phase response that, similar to measuring CRP in humans, can be quantified by measuring the acute phase protein serum amyloid A (SAA). SAA is produced by the liver upon stimulation by proinflammatory cytokines such as IL-1, IL-6, and TNF-$\alpha$.

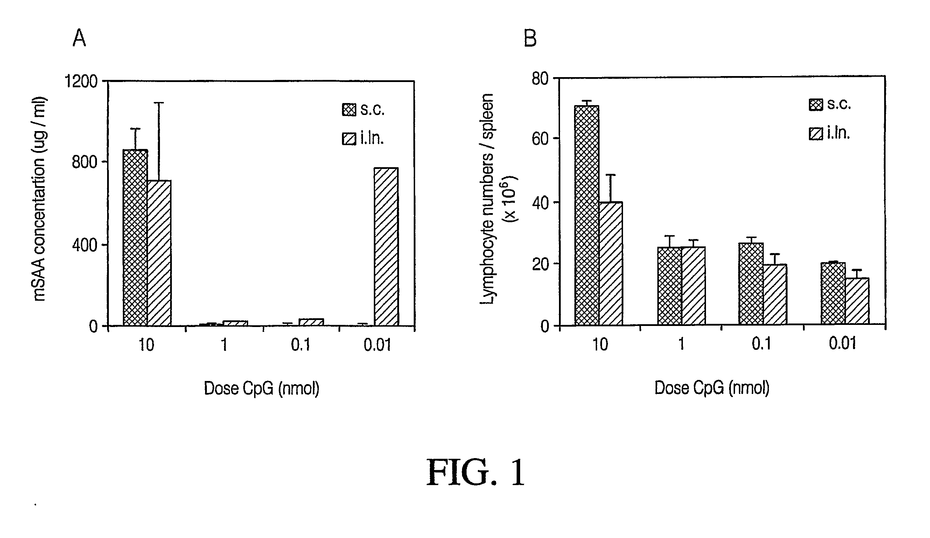

Mice were subcutaneously (s.c.) or intralymphatically (i.1n.) injected with titrated amounts of CpG ODN 1668pt (**SEQ. ID. No. 1**) (10, 1, 0.1 and 0.01 nmol) and 24 hours later analyzed for the presence of enhanced concentrations of the acute phase protein serum amyloid A (mSAA) in the blood. mSAA levels were assessed by ELISA. Elevated serum levels of SAA were observed in mice receiving CpG doses of 10 nmol, regardless of the route of administration. CpG doses lower than 1 nmol did not induce a measurable acute phase response (Figure 1 A). The means $\pm$ SEM (n = 3) are shown. These results show that irrespective of the administration route, an acute phase reaction was observed only at the highest dose of CpG (10 nmol).

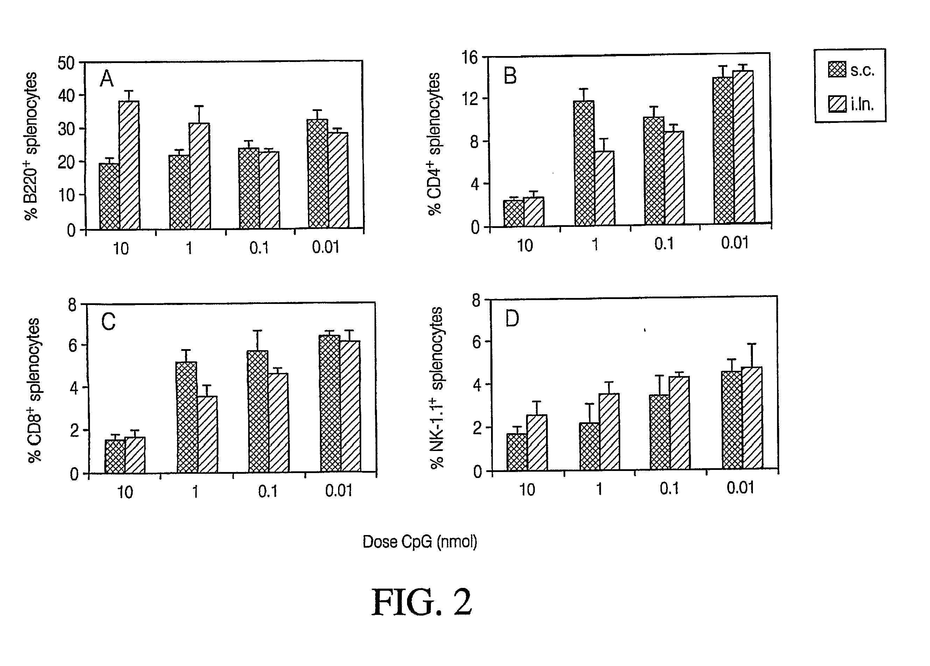

Since the administration of CpGs at doses of 10 - 20 nmol has been reported to induce splenomegaly with changes in cell composition (Sparwasser, T., L. et al., "Immunostimulatory CpG-oligodeoxynucleotides cause extramedullary murine hemopoiesis," J Immunol 162:2368, 1999 (which is incorporated herein in its entirety)) these changes were assessed as an additional parameter of adverse effects. C57BL/6 mice were treated with different doses of CpG and euthanized 8 days later for analysis of the spleen. Similar to mSAA levels, splenomegaly and changes in cell composition were observed only with the high doses of CpG (Figure 1 B). The means $\pm$ SEM (n = 3) are shown. At 10 nmole, splenocyte numbers increased about 2—3-fold for both immunization routes. Only moderate changes were observed for B lymphocytes (Figure 2 A). In contrast, the relative amounts of CD4 and CD8 T cells as well as NK cells were substantially reduced in a dose-dependent manner for both the subcutaneous and intralymphatic route of CpG administration (Figure 2 B-D). The means $\pm$ SEM (n = 3) are shown. Hence, these results confirm that administration of CpG shows considerable adverse effects at the "recommended" doses around 10 nmol for ODN 1668pt, whereas 0.1 and 0.01 nmol did not induce any detectable side effects.

Example 2

Intralymphatic administration of CpG enhances activation of dendritic cells.

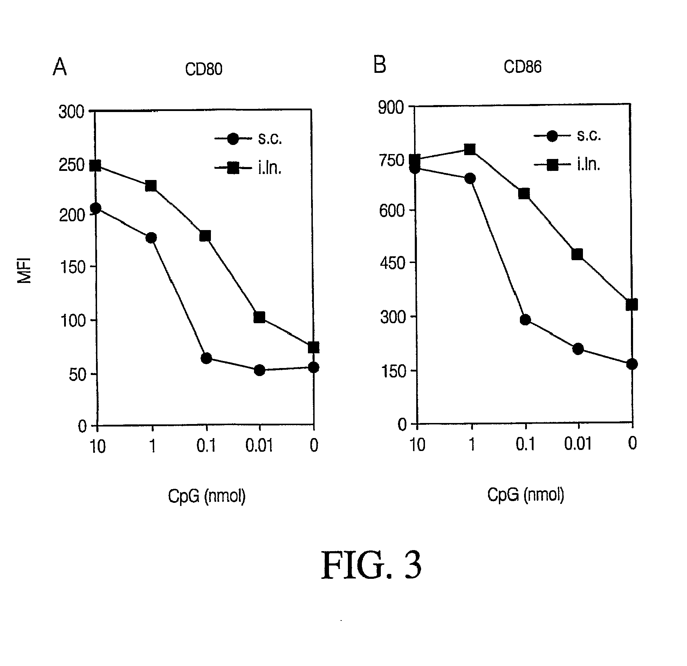

To assess CpG-induced activation of professional APCs, C57BL/6 mice received titrated amounts of CpG ODN 1668pt either subcutaneously by injection into the inguinal region or by direct injection into the inguinal lymph node. After one day the inguinal lymph nodes were collected and DCs were isolated by Collagenase D digestion and positive isolation using anti-CD11c magnetic beads. Activation of DCs was assessed by measuring up-regulation of CD80 and CD86 (Figures. 3A and B). Values represent the mean fluorescence intensity (MFI) measured by flow cytometry. As control, mice were immunized i.1n. with saline solution. Lymph node cells pooled from three mice per group were used for the analysis. At least $1.5 \times 10^4$ CD11c$^+$ cells were acquired per condition. High CpG doses of 10 and 1 nmol significantly enhanced expression of CD80 and CD86, regardless of the route of administration. At low CpG doses of 0.1 and 0.01 nmol, however, only direct injection into the inguinal lymph node induced significant DC maturation. Note that an intralymphatic control injection with saline induced slight upregulation of CD80 and CD86. This may be explained by the physical lymph node damage produced by the needle. Thus, although both sites of injection are very close to each other, direct intralymphatic administration reduced the dose of CpG that is necessary for DC maturation by 10—50 fold.

Example 3

Cytotoxicity induced by ovalbumin together with low amounts of CpG ODN (0.01 nmol) given intralymphatically.

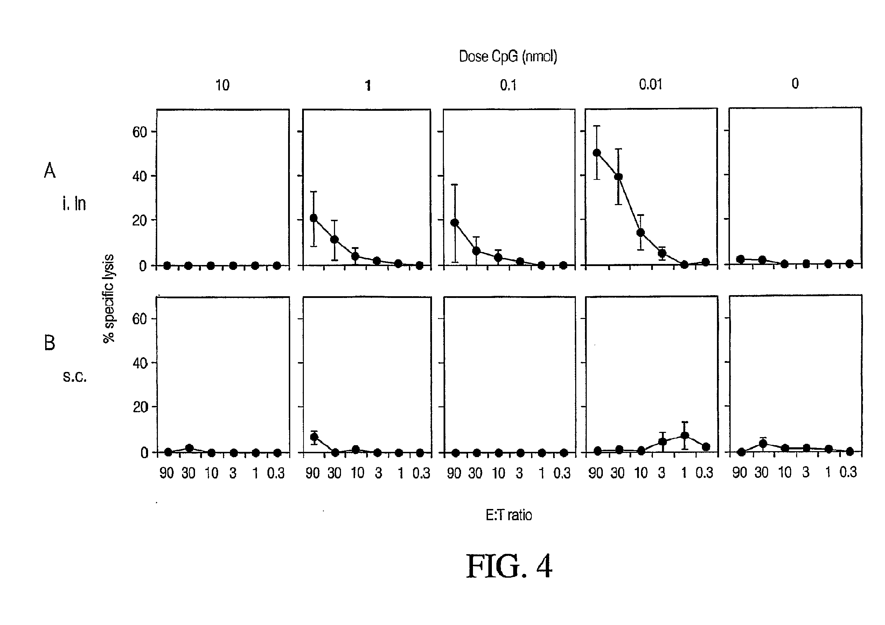

The optimal intralymphatic dose of CpG for CTL induction was evaluated and compared to the optimal subcutaneous dose. C57BL/6 mice were injected with OVA alone or in combination with CpG ODN 1668pt. CD8 T cell responses were tested for direct *ex vivo* CTL activity seven days later using $^{51}$Cr-release assays on EL-4 target cells pulsed with the ovalbumin peptide epitope (**SEQ. ID. No. 2**) at various effector to target cell ratios. Two dilution series of effector cells per mouse were performed. The means $\pm$ SEM (n = 3) are shown (see Figure 4). While mice vaccinated intralymphatically showed significant induction of cytotoxic CD8 T cells even at 0.01 nmol CpG, subcutaneous vaccination did not elicit CTL activity in this assay system. The CTL activity was correlated with the frequency of CD8 lymphocytes staining positive for intracellular IFN-$\gamma$ as measured by flow cytometry (data not shown). These data demonstrate a potent generation of cytotoxic immunity against a dominant epitope borne by a model protein antigen, by intralymph node co-administration of CpG ODNs at doses that were not associated with systemic acute phase reaction.

Example 4

Intranodal delivery of antigen plus low dose CpG ODN or polyI:C increases a CD8$^{\pm}$ T cell response against a tumor-associated antigen

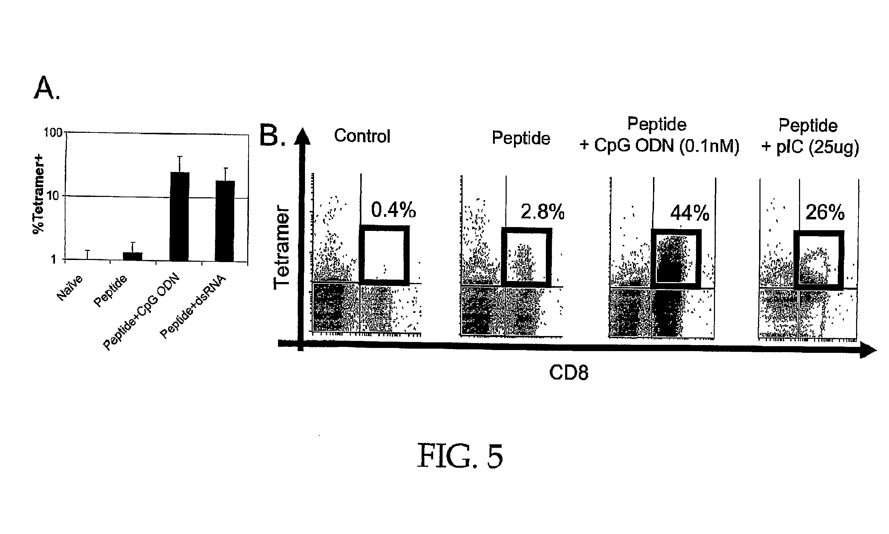

H-2 class I-negative, HLA-A2.1-transgenic (HHD-1) mice (Pascolo, S., et al., J Exp Med. 185:2043-2051, 1997 (which is incorporated herein in its entirety)) were housed under pathogen-free conditions and used for evaluation of the immunogenicity of HLA-A2.1-restricted human tumor-associated cytotoxic T lymphocyte (CTL) epitopes. Female mice 8-12 weeks of age were used for intralymphatic immunization and for isolation of splenocytes for *in vivo* cytotoxicity studies. HHD-1 mice were immunized with 25 $\mu$L of a 1 mg/mL solution in PBS of Melan-A peptide (**SEQ. ID. No. 3**) alone, plus 0.1 nmole of a CpG ODN (Sigma) (**SEQ. ID. No. 4**), or plus 25$\mu$g polyI:C, a synthetic double stranded RNA, by injection, bilaterally, into the inguinal lymph nodes. A total of four rounds of injections were given, on days 0, 3, 14 and 17.
The immune response was measured by tetramer and CD8 double staining of splenocytes from the immunized mice. Mononuclear cells isolated from peripheral blood after density centrifugation (Lympholyte Mammal, Cedarlane Labs) with HLA-A*0201 MART1 (**SEQ. ID. No. 3**)-PE MHC tetramer (Beckman Coulter) and FITC conjugated rat anti-mouse CD8a (Ly-2) monoclonal antibody (BD Biosciences). Data was collected using a BD FACS Calibur flow cytometer and analysed using Cellquest software by gating on the lymphocyte population and calculating the percent of tetramer$^+$ cells within the CD8$^+$ CTL population. The results (see Figure 5) were expressed as means $\pm$ SEM of % tetramer stained cells within the CD8+ T cell population (A), and with specific dot plot plots representative for each group (B). Splenocytes from naive transgenic mice were included as controls. The data show that co-administration of a peptide with a dose of CpG ODN that is not associated with systemic acute phase reaction, nor with observed mortality or morbidity, results in potent immune response against a tumor-associated antigen. Similarly, use of polyI:C, at doses not associated with observed mortality or morbidity, was also capable of supporting induction of a robust antigen specific response.

Example 5

Intranodal delivery of low dose BRM plus antigen induces effective CTL immunity against a tumor-associated antigen.

The effectiveness of CTL generated as described in Example 4 was assessed as *in vivo* clearance of tumor cells from pulmonary tissue in mice challenged with Melan-A$^+$ tumor cells. Human melanoma tumor target cells, 624.38 (Melan-A$^+$, HLA-A2$^+$) were cultured in RPMI medium supplemented with 10% fetal bovine serum (Hyclone), 0.1mM non-essential amino acids, and 0.3 mg/mL L-glutamine in a 5% CO$_2$ incubator at 37°C. The HLA-A2$^-$ subclone, 624.28, was grown under the same conditions and served as a negative control. Specific targets were prepared by incubating 10$^8$ 624.38 cells with 2.5 $\mu$M CFSE in PBS for 20 minutes (CFSE$^{hi}$) and 10$^8$ HLA-A2$^-$, 624.28 target controls were labelled with 0.25 $\mu$M CFSE (CFSE$^{lo}$). Each immunized HHD-1 mouse was intravenously injected in the tail vein with a mixture of 10$^7$ CFSE$^{hi}$ 624.38 and 10$^7$ CFSE$^{lo}$ 624.28 cells followed by an identical injection 3 hours later. Un-immunized HHD-1 mice were also injected and served as naïve controls. After 14 hours, peripheral blood was harvested via cardiac puncture and the mononuclear cells were isolated by density centrifugation and stained with tetramer as described above. The lungs were surgically removed, minced, and treated with 0.1 % collagenase buffer for 2 hours. The mononuclear cells were then isolated by density centrifugation and CFSE positive cells were analysed by FACS. The *in vivo* specific lysis of human melanoma target cells in the lung was calculated by the following formula:

$$[(1-\%CFSE^{hi} / \%CFSE^{lo}) - (1- \%CFSE^{hi} \text{ Control} / \%CFSE^{lo} \text{ Control})]$$

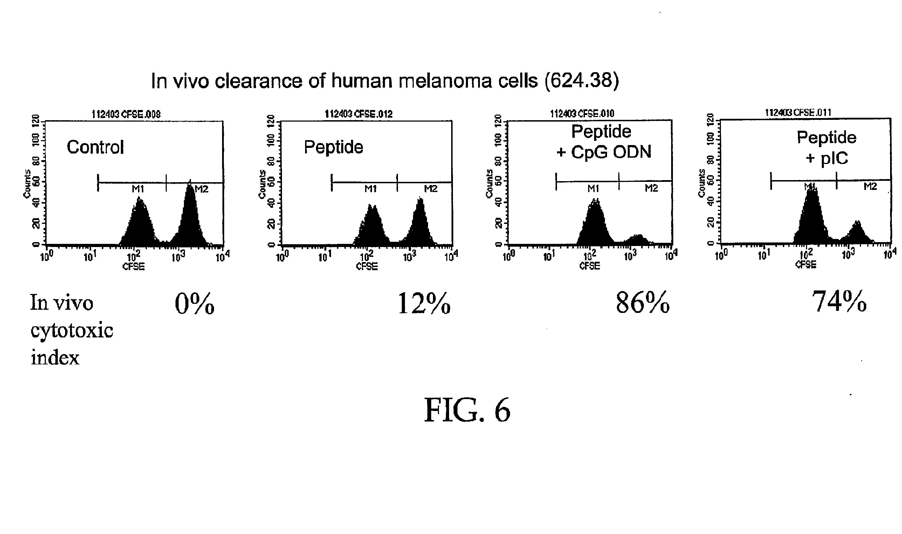

The histograms shown in Figure 6 were representative for the three experimental groups. Immunization with antigen alone, though generating a readily detectable response by tetramer staining, resulted in only minimal tumor cell lysis. In contrast inclusion of a BRM in the protocol led to substantial tumor cell lysis. Thus co-administration of a peptide with a dose of CpG ODN that is not associated with systemic acute phase reaction, nor with observed mortality or morbidity, results in potent cytolytic response against human tumor cells. Similarly, use of polyI:C, at doses not associated with observed mortality or morbidity, was also capable of supporting induction of substantial lytic activity.

Example 6.

Induction of cytotoxic immunity by co-administration of defined self or non-self MHC class I restricted antigen plus adjuvant into the lymph nodes.

HHD-A2 transgenic mice (n=4/group) were immunized with various peptides (see Table 1) admixed with polyI: C, by direct inoculation into the inguinal lymph nodes (12.5μg peptide + 12.5μg of adjuvant, in 25μl of PBS / each inguinal lymph node at day 0, 3, 14 and 17). One week after the final administration, splenocytes were stimulated *ex vivo* with 10μg/ml of peptide in presence of 5U/ml of rIL-2 and tested in a standard cytotoxic assay, against $^{51}$Cr-labeled target cells (T2 cells) uncoated or coated with cognate peptide, at various Effector:Target ratios. Alternatively, in the case of PSMA 288-297 epitope, cytotoxicity was measured against PSMA expressing human tumor cells (LNCap) cells coated or not with 10μg/ml of MHC class I blocking antibody (W6/32). Cytotoxicity resulting from immunization with the NY-ESO-1 157-165 epitope was also assessed against 624.28 and 624.38 cells that had been transformed to express a GFP/NY-ESO-1 fusion protein.

Table 1

| Antigen (peptide) | Identity in mouse |
|---|---|
| Hu-PSMA 288-297 **SEQ. ID. No. 5** | Self: conserved between mouse and human |
| Hu-PSMA 730-739 **SEQ. ID. No. 6** | Non-self: not conserved between mouse and human |
| Hu-PRAME 425-433 **SEQ. ID. No. 7** | Non-self: not conserved between mouse and human |
| Hu-PRAME 300-309 **SEQ. ID. No. 8** | Non-self: not conserved between mouse and human |
| Hu-NY-ESO-1 157-165 **SEQ. ID. No. 9** | Non-self: not conserved between mouse and human |

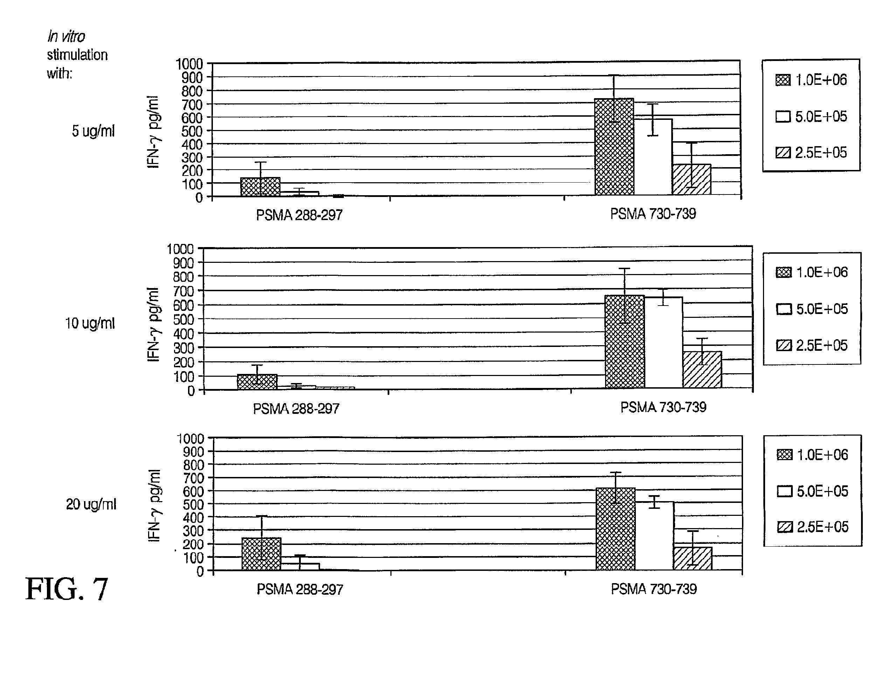

Alternatively, mice were immunized with the PSMA 288-297 or PSMA 730-739 epitope peptide in IFA and peptide by subcutaneous injection (5μg peptide in 100μl, twice, at day 0 and 7). One week after the last administration, splenocytes were stimulated *ex vivo* with 5μg (Figure 7, top panel), 10μg (Figure 7, middle panel) and 20μg/ml (Figure 7, bottom panel) of peptide for 3 days and the IFN-gamma concentration in cell supernatants measured by ELISA.
The results show that irrespective of the self or non-self nature of the epitope (reflected in the overall diminished responsiveness to self epitope by a conventional immunization procedure — Figure 7), co-administration of peptide and polyI:C (synthetic dsRNA) into the lymph nodes triggered a specific cytotoxic response recognizing target cells pulsed with peptide (Figure 8A-C and left half of D) or tumor cells expressing the target antigen (Figure 8E and right half of D).

Example 7

Enhanced IgG antibody responses agaisnt PLA2 by intra lymph node co-administration of CpG ODN and antigen.

The doses of CpG required to enhance Th1-dependent IgG immune responses against an allergen, and whether intralymphatic administration could reduce the required dose of CpG were evaluated. PLA2, the major allergen from bee venom, was used as a model allergen. CBA/J mice were immunized biweekly with 10, 1, 0.1 and 0.01 nmol ODN-1668pt together with 0.1 μg of PLA2 either intralymphatically (A) or subcutaneously (B). Three weeks after the third injection, sera of mice were collected and PLA2-specific IgG was determined by ELISA (Figure 9). Values represent means and standard deviations obtained from three mice per group. One representative of two similar experiments is shown. In subcutaneously immunized mice, the highest IgG titers were induced by using CpG doses of 10 and 1 nmol, which is consistent with the doses described in the literature (Krieg, A. M., "CpG motifs in bacterial DNA and their immune effects," Annu Rev Immunol 20:709, 2002 (which is incorporated herein in its entirety)). If CpG doses were lowered to 0.1 and 0.01 nmol an adjuvant effect was no longer detectable. The picture was reversed for the intralymphatic immunization route, where highest antibody titers were observed for low CpG doses of 0.1 and 0.01 nmol and no antibody responses were detectable with the high CpG dose of 10 nmol. Note that PLA2 given alone did not generate significant IgG titers (not shown). These data demonstrate that subcutaneous administration of CpG required significantly higher doses than intralymphatic administration. Importantly, the optimal adjuvant effect of CpG for intralymphatic immunization was achieved at doses that did not cause detectable systemic side effects.

This example shows a potent generation of specific IgG antibodies against PLA2 by intra lymph node co-administration of CpG ODNs, at lower doses that were not associated with systemic acute phase reaction.

## Example 8

Enhanced IgG antibody responses against PLA2 by intra lymph node co-administration of various TLR-ligands and antigen.

To evaluate the effect of various TLR-ligands on the antibody response, CBA/J mice were immunized with three injections at two week intervals of 0.1 μg phospholipase A2 (PLA2) and a TLR ligand; specifically S. *aureus* peptidoglycan (PGN), *E.coli* lipopolysaccharide (LPS), polyriboinosinic polyribocytidylic acid (PolyI:C), lipoteichoic acid (LTA), flagellin, phosphorothioate-modified CpG ODN 1826 **(SEQ. ID. No. 10)**, and the experimental TLR-7/8-binding compound 3M003 (a gift from 3M Corporation, St. Paul, MN) (see table 2). The compounds were mixed within an hour before injection into the inguinal lymph node.

Table 2. Description of adjuvants used for immunizations

| Adjuvant | Dose | TLR-L number | Description & Source |
|---|---|---|---|
| LTA | 1.25 μg | 2 | Lipoteichoic acid from St aureus |
| PGN | 2 μg | 2 & 6 | Peptidoglycan from St aureus |
| LPS | 2 μg | 2 & 4 | Lipopolysaccharide from E. coli |
| Poly(I:C) | 2 μg | 3 | Synthetic analogue of dsRNA |
| Flagellin | 3 μg | 5 | S. thyphimurium |
| 3M003 | 2 μg | 7 & 8 | Small synthetic antiviral imidazoqionoline |
| CpG | 1 nmole | 9 | Synthetic dsDNA with repeating C and G motives |
| Al(OH)$_3$ | 60 μg * | - | Aluminium hydroxide |

\* injected amount with respect to Al$^{3+}$

Figure 10 illustrates PLA2-specific IgG2a and IgG1 antibodies in sera as well as the ratio of IgG2a to IgG1 titers (determined by ELISA) at different time points (Fig. 10); the ratio of IgG2a to IgG1 antibodies in murine serum is typically used as a qualitative measure for the relative strength of Th1 (IgG2a) and Th2 (IgG1) type immune responses. The sera were obtained over 13 weeks and analyzed by ELISA for anti-PLA2 IgG2a (Fig. 10A) and IgG1 (Fig. 10B). The ratio of IgG2a to IgG1 titers was calculated as a measure for Th1/Th2 immune response balance (Fig. 10C). The serological titers were defined as the inverse of the highest dilution yielding an absorbance higher than that of a negative serum plus three standard deviations and expressed as geometric means $\pm$ standard error (n=3-4). Pre-immunization levels of anti-PLA2 antibodies were measured on sera from five mice taken the day before first injection. The results from one representative out of two immunization experiments are shown.

Seven weeks after the first and three weeks after last injection notable sero-conversion was observed in mice receiving LPS, PolyI:C or CpG. CpG induced IgG2a (Fig. 10A), LPS predominantly IgG1 (Fig. 10B), and PolyI:C induced high antibodies titers of both IgG1 and IgG2a isotypes. This is also highlighted in the resulting isotype ratios (Fig. 10C). Other TLR ligands tested also showed IgG1 and IgG2a antibodies in early sera compared to background levels. When PLA2 was administered together with the conventional adjuvant aluminium hydroxide, a balanced profile of IgG2a and IgG1 antibodies was produced, but the titers were lower than those produced by PolyI:C- or LPS-containing vaccines. Also, the ratio of IgG2a to IgG1 was shifted towards Th2 when compared to PolyI:C, CpG and peptidoglycan (Fig. 10C).

None of the vaccines induced detectable IgE antibodies against PLA2 upon intralymphatic injection (data not shown).

The kinetic of the antibody production was also affected by the adjuvant. PolyI:C produced its maximum levels of antibodies within three weeks of the final boost injection (seven weeks after initiating immunization). LPS caused a delay of three weeks for both the IgG1 and the IgG2a isotypes, and the IgG2a titer increased relatively more than the IgG1. In seven-week sera from CpG-injected mice, IgG1 was hardly detectable but increased at 10 and 13 weeks indicating a delay in the IgG1 antibody production. However, the serum concentration of IgG1 was geometrically only 30-50% of the titers induced by LPS or PolyI:C. As with LPS, peak titers were delayed when peptidoglycan and flagellin were used as the BRM. Peptidoglycan induced predominantly IgG2a, whereas flagellin induced both IgG2a and IgG1.

Example 9.

Modulated Cellular Responses against PLA2 by intra lymph node co-administration of various TLR-ligands and antigen.

After the last bleeding (ten weeks after the third injection), mice from selected groups described in Example 8, above, (aluminium hydroxide, PolyI:C, LPS, CpG and 3M003) received another boost injection of PLA2 and adjuvant. Two weeks later spleen cells were isolated, and analysed directly for intracellular IFN-γ by flow cytometry (Fig. 11) or analyzed for IFN-γ, IL-4 and IL-10 secretion by ELISA after four days cultivation with 10 μg/ml PLA2 (Fig. 12). For Figure 11, intracellular IFN-γ was measured by flow cytometry after four hours stimulation of cells with PdBu and ionomycin (both at 500 ng/ml) in the presence of brefeldin A (10μg/ml). The cells were then incubated with anti-CD16/CD32 for Fc-recptor blocking and stained on ice and in PBS/FCS 2% with fluorescent-labelled Abs against CD4, CD8 and CD44 (all Abs from BD Biosciences (Franklin Lakes, NJ). Cells were subsequently fixed with 4% paraformaldehyde and permeabilised with 0.1% Nonidet P-40 before staining with Ab against IFN-γ. Four-color cytometry were performed on a FACSCalibur flow cytometer (BD Biosciences, San Jose, CA). Cells were gated on CD4 or CD8 positive lymphocytes and the results expressed as means ($\pm$ S.D.) for three mice, as percent of T-cell subset (A) or as absolute numbers in the spleen (B). One representative out of two immunization experiments is shown. For Figure 12, after homogenisation of spleens and lysis of red blood cells, $5x10^5$ splenocytes were cultured with 20 μg/ml PLA2 protein in 200 μl IMDM (Gibco) supplemented with L-glutamine (4 mM), FCS (10%), mercaptoethanol (75 μM), as well as streptomycin and ampicillin. Supernatants were collected after 96 hours incubation and IL-4, IL-10 and IFN-γ concentrations determined by ELISA from R&D Systems (Abingdon, United Kingdom). Cytokine concentrations in supernatants as determined by ELISA after subtraction of spontaneous cytokine secretion was determined upon *in vitro* stimulation with an irrelevant antigen (ovalbumin). Statistical differences between the different groups were indicated when significant (P < 0.01). One representative out of two immunization experiments is shown.

A higher percentage of IFN-γ producing cells were elicited from CD8-positive T cells (Fig. 11B) than from CD4-positive T cells (Fig. 11A), with CpG showing the strongest effect. The same was evident with regard to the absolute number of cytokine-producing cells (Fig. 11C-D).

The capacity to produce IFN-γ correlated with the amount of cytokine secreted as measured by ELISA. The highest amounts of IFN-γ were produced by cells from mice immunized using PolyI:C and CpG (Fig. 12A), the concentrations measured being significantly higher than those obtained from mice immunized with LPS or 3M003 (P < 0.01). Interestingly, whereas only marginal anti-PLA2 antibodies could be detected after immunizing mice with PLA2 and 3M003, this vaccine regime apparently generated a substantial number of IFN-γ producing cells as observed both in FACS (Fig. 11) and in ELISA (Fig. 12). CpG caused significantly reduced IL-4 secretion (Fig. 11B) when compared with 3M003 and PolyI:C (P<0.01) or LPS (P<0.05), despite triggering the strongest IL-10 production (Fig. 12C).

Example 10

Imiquimod exhibits adjuvant activity when administered intranodally in amounts ineffective when administered subcutaneously.

Imiquimod, 1-(2-methylpropyl)-1H-imidazol [4,5-c] quinolin-4-amine, is a topical immune response modifier that is approved for the use in the treatment of human genital warts. Imiquimod binds to TLR-7 in mice and TLR-7 and TLR-8 in humans. Such Imidazoquinoline binding leads to activation of plasmacytoid dendritic cells (DCs). Subsequent rapid maturation of the DCs is associated with upregulation of expression of several DC cell surface components, including the co-stimulatory molecules CD40, CD80, and CD86, as well as MHC class I and II molecules, and chemokine receptors. TLR binding also stimulates the production of a characteristic profile of proinflammatory cytokines including IL-1, IL-6, IL-12, interferon-γ, and TNF-α. These characteristics make Imiquimod a very potent adjuvant candidate. Not surprisingly, however, Imiquimod shows significant toxicity when systemically administered, including fatigue, malaise, fever, headache, and lymphocytopenia (Witt, P.L. et al., Cancer Res. 53(21):5176-80, 1993 (incorporated herein by reference in its entirety)). Even if only topically used on the skin of children in a manner that allows for enhanced penetration, fever may be observed (Campanelli, A. et al. J Am Acad Dermatol. 52(1):E1, 2005 (incorporated herein by reference in its entirety)).

C57BL/6 mice were immunized with the immunodominant MHC class I binding epitope of the lymphocytic choriomeninigitis virus glycoprotein, p33 (SEQ. **ID. No. 11**). On day 8 after immunization mice were bled to analyze p33-specific CD8+ T cell function by FACS staining of CD8, CD44 and intracellular interferon-γ production after a 4-hour in vitro stimulation with p33. All groups of mice received the same dose of 10 μg p33. The adjuvant Imiquimod, however, was administered by different routes and in different doses. Mice were either injected intralymphatically with 250μg or 25μg of Imiquimod, or they were injected subcutaneously with 250μg or 25μg of Imiquimod. Control mice were immunized with p33, but received no Imiquimod. As seen in Figure 13, 250μg of Imiquimod enhanced induction of p33-specific CD8+ T cells only, when administered intralymphatically, but not after subcutaneous administration. Thus, intralymphatic administration of Imiquimod enhanced its adjuvant activity.

Without being bound by any particular model, the foregoing examples can be understood as resulting from the co-localization of antigen and immune modulator within the same microenvironment. In this view, very low amounts of immune modulator are associated with extremely high ratios between local and systemic (non-lymphatic) bioavailability of such compounds when delivered into lymphoid organs, resulting in substantial local effect with minimal systemic impact. *See* Figure 14. More specifically, intralymph node delivery of minute amounts of BRM results in appropriate local bioavailability and immune activity since the lymphoid organs are the primary location where the immune response is initiated or amplified (Figure 14, upper row diagrams). In contrast, delivery of such minute amounts of BRM via intramuscular, subcutaneous or intravenous route, will result in reduced localization of BRM into lymphoid organs, simply because systemic clearance mechanisms or PK profile will play a more prominent role (Figure 14, lower row diagrams). Thus, to achieve a similar potency, more BRM needs to be administered via a conventional route (subcutaneous, intramuscular). Since the toxicity profile is dependent on the dosage, targeted intralymphatic delivery result in an overall improved therapeutic index.

Thus, sub-nanomole doses of CpG ODN were extremely effective in promoting immunity against a tumor associated antigen, Melan-A, when the delivery route was intralymphatic. On average, in A2-transgenic mice immunized with the CpG peptide cocktail, approximately one out of five CD8+ T cells were tetramer positive - which represents a considerable expansion of the specific CD8+ T cell population. Peptide immunization alone was not very effective in inducing a robust T cell population expansion, even when its inherently poor pharmacokinetics profile was mitigated by intralymphatic delivery; this may be due to limited co-expression of immune activating receptors on APC in the absence of adjuvant usage. Notably, the co-administration of CpG ODN obviated the need for Th response in the process of induction of anti-tumoral immunity. In addition, it resulted in effective surveillance of non-lymphatic organs, as exemplified by the significant clearance of A2$^+$ melanoma target cells in transgenic mice previously immunized via the intranodal route.

In various embodiments, numbers expressing quantities of ingredients, properties such as molecular weight, reaction conditions, and so forth used to describe and claim certain embodiments of the invention can be understood as being modified by the term "about." Accordingly, in some embodiments, the numerical parameters set forth in the written description and attached claims are approximations that may vary depending upon the desired properties sought to be obtained by a particular embodiment. In some embodiments, the numerical parameters can be construed in light of the number of reported significant digits and by applying ordinary rounding techniques. The numerical values presented in some embodiments of the invention may contain certain errors necessarily resulting from the standard deviation found in their respective testing measurements.

In various embodiments, the terms "a" and "an" and "the" and similar referents used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. Methods described herein can be performed in any suitable order unless otherwise indicated herein or otherwise clearly contradicted by context. The use of any and all examples, or exemplary language (e.g. "such as") provided herein is intended merely to better illuminate the invention and does not pose a limitation on the scope of the invention otherwise claimed. No language in the specification should be construed as indicating that any non-claimed element is essential to the practice of the invention.

Groupings of alternative elements or embodiments of the invention disclosed herein are not to be construed as limitations. Each group member may be referred to and claimed individually or in any combination with other members of the group or other elements found herein. It is contemplated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of this invention are described herein, including the best mode known to the inventors for carrying out the invention. Variations on those preferred embodiments will become apparent to those of ordinary skill in the art upon reading the foregoing description. It is contemplated that skilled artisans can employ such variations as appropriate, and the invention can be practiced otherwise than specifically described herein. Accordingly, many embodiments of this invention include all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification. Each of the above cited references and printed publications are herein individually incorporated by reference in their entirety.

Accordingly, it is to be understood that the embodiments of the invention disclosed herein are illustrative of the principles of the present invention. Other modifications that can be employed are within the scope of various embodiments of the invention. Thus, by way of example, but not of limitation, alternative configurations of the present invention may be utilized in accordance with the teachings herein. Accordingly, the present invention is not limited to that precisely as shown and described.

Some embodiments of the invention are disclosed in the following items.

Items

1. A method of improving the therapeutic index of a BRM, the method comprising the step of: administering a BRM to a secondary lymphoid organ of a subject in a lymphatically effective dose wherein the lymphatically effective dose avoids or reduces a BRM-related adverse clinical event as compared to use of a non- lymphatically effective dose of the BRM.

2. The method of item 1 wherein the modulated immune response is antigen-specific.

3. The method of item 1 wherein little or no BRM-related adverse clinical event results.

4. The method of item 1 wherein a lymphatically effective dose is at least 10-fold less than the non-lymphatically effective dose.

5. The method of item 2 comprising co-administration of the antigen with the BRM.

6. The method of item 2 comprising administering the antigen proximal in time to the BRM

7. The method of item 1 comprising, administration to an identified area of relatively high lymphatic drainage.

8. The method of item 2 comprising administering the antigen to an identified area of high lymphatic drainage that drains to the secondary lymphoid organ of the subject.

9. The method of item 2 wherein the antigen is endogenously present in the secondary lymphoid organ of the subject.

10. The method of item 1 comprising administration directly to a lymph node or lymph vessel.

11. The method of item 2 wherein the modulated immune response comprises an increased response to the antigen.

12. The method of item 2 wherein the modulated immune response comprises a decreased response to the antigen.

13. The method of item 2 wherein the modulated immune response comprises a shift toward a humoral response to the antigen.

14. The method of item 1 wherein the modulated immune response comprises a shift in [the] relative proportions of individual antibody isotypes.

15. The method of item 2 wherein the modulated immune response comprises a shift toward a cellular response to the antigen.

16. The method of item 1 wherein the modulated immune response comprises a shift toward a T1 response.

17. The method of item 1 wherein the modulated immune response comprises a shift toward a T2.

18. The method of item 15 wherein the modulated immune response comprises a shift toward a CTL response to the antigen.

19. The method of item 15 wherein the modulated immune response comprises a shift toward a T helper cell response to the antigen.

20. The method of item 15 wherein the modulated immune response comprises a shift toward a T regulatory cell response to the antigen.

21. The method of item 1 wherein the BRM comprises a cytokine or chemokine.

22. The method of item 1 wherein the BRM comprises a toll-like receptor ligand.

23. The method of item 22 wherein the BRM comprises ds RNA.

24. The method of item 22 wherein the BRM comprises a DNA comprising a CpG sequence.

25. The method of item 24 wherein the DNA comprises an oligonucleotide.

26. The method of item 24 wherein the DNA comprises a plasmid.

27. The method of item 22 wherein the toll-like receptor ligand is selected from the group consisting of peptidoglycan, LPS, LPS analogues, flagellin, lipoteichoic acid, an imidazoqionoline, imiquimode, resiquimod, microbial nucleic acids, CpG-containing oligonucleotides, double-stranded RNA, and polyI:C.

28. The method of item 1 wherein the BRM comprises a small molecule, antibody, or engineered soluble ligand that acts as an agonist or an antagonist specific for a target selected from the group consisting of cellular receptors, co-stimulatory receptors, cytokine receptors, chemokine receptors, signal transduction elements, and transcriptional regulators.

29. The method of item 2 wherein the antigen comprises a tumor-associated antigen.

30. The method of item 2 wherein the antigen comprises a microbial antigen.

31. The method of item 30 wherein the microbial antigen is associated with a protist, fungi, bacterium, virus, or prion.

32. The method of item 2 wherein the antigen comprises an allergen, a toxin, or toxoid.

33. The method of item 2 wherein the antigen comprises a disease-matched antigen.

34. A method of modulating an antigen-specific immune response, the method comprising the step of:

administering a BRM to a secondary lymphoid organ of a subject in a dose sufficient to obtain a modulated immune response.

35. A method of modulating an immune response to an antigen comprising the step of:

co-administering a BRM and said antigen to a secondary lymphatic organ whereby the antigen-specific immune response is modulated, wherein the modulation does not consist essentially of an augmented CTL response.

36. The use of a BRM in the manufacture of a medicament suitable for administration to a secondary lymphoid organ of a subject.

37. The use of a BRM in the manufacture of a medicament suitable for modulating an immune response in a subject without causing a severe BRM-related adverse clinical event.

38. The use of a BRM in the manufacture of a medicament that increases the therapeutic index of the BRM and that is suitable for modulating an immune response in a subject.

39. The use of a BRM and an antigen in the manufacture of a medicament for modulating an immune response in a subject that is specific to said antigen and that is suitable for injection into a secondary lymphoid organ of a subject.

40. A composition comprising a lymphatically effective dose of a BRM, wherein said lymphatically effective dose comprises an amount of a BRM that is relatively nontoxic and wherein said lymphatically effective dose is less than a non- lyphatically effective dose.

41. A composition comprising a lymphatically effective dose of a BRM, wherein said lymphatically effective dose comprises an amount of a BRM that is relatively non-toxic and wherein the amount of the BRM is insufficient to be a non- lyphatically effective dose.

42. A composition comprising a lymphatically effective dose of a BRM and an antigen, wherein said lymphatically effective dose comprises an amount of a said lymphatically effective dose comprises an amount of a BRM that is relatively non-toxic and wherein the amount of the BRM is insufficient to be a non-lyphatically effective dose.

43. A composition comprising a lymphatically effective dose of a BRM wherein said lymphatically effective dose of a BRM is at least ten-fold less than the corresponding non-lyphatically effective dose.

**Claims**

1. A method of improving the therapeutic index of a BRM, the method comprising the step of: administering a BRM to a secondary lymphoid organ of a subject in a lymphatically effective dose wherein the lymphatically effective dose avoids or reduces a BRM-related adverse clinical event as compared to use of a non-lymphatically effective dose of the BRM.

2. The method of claim 1 wherein the modulated immune response is antigen-specific.

3. The method of claim 1 wherein little or no BRM-related adverse clinical event results.

4. The method of claim 1 wherein a lymphatically effective dose is at least 10-fold less than the non-lymphatically effective dose.

5. The method of claim 2 comprising co-administration of the antigen with the BRM.

6. The method of claim 2 comprising administering the antigen proximal in time to the BRM

7. The method of claim 1 comprising administration to an identified area of relatively high lymphatic drainage.

8. The method of claim 2 comprising administering the antigen to an identified area of high lymphatic drainage that drains to the secondary lymphoid organ of the subject.

9. The method of claim 2 wherein the antigen is endogenously present in the secondary lymphoid organ of the subject.

10. The method of claim 1 comprising administration directly to a lymph node or lymph vessel.

11. The method of claim 2 wherein the modulated immune response comprises an increased response to the antigen.

12. The method of claim 2 wherein the modulated immune response comprises a decreased response to the antigen.

13. The method of claim 2 wherein the modulated immune response comprises a shift toward a humoral response to the antigen.

14. The method of claim 1 wherein the modulated immune response comprises a shift in [the] relative proportions of individual antibody isotypes.

**15.** The method of claim 2 wherein the modulated immune response comprises a shift toward a cellular response to the antigen.

FIG. 1

EP 2 351 577 A1

FIG. 2

FIG. 3

EP 2 351 577 A1

FIG. 4

FIG. 5

In vivo clearance of human melanoma cells (624.38)

|            | Control | Peptide | Peptide + CpG ODN | Peptide + pIC |
| ---------- | ------- | ------- | ----------------- | ------------- |
| In vivo cytotoxic index | 0% | 12% | 86% | 74% |

FIG. 6

FIG. 7

FIG. 8A

EP 2 351 577 A1

FIG. 8B

EP 2 351 577 A1

FIG. 8C

FIG. 8D

FIG. 8E

EP 2 351 577 A1

FIG. 9

Isotype profile of anti-PLA2 serom antibodies.

FIG. 10

Intracellular IFN-$\gamma$ in CD4[+] and CD8[+] T cells.

FIG. 11

Secretion of cytokines in vitro

FIG. 12

IIntracellular IFN-$\gamma$ staining of CD44$^+$ CD8$^+$ cells following administration of imiquimod.

FIG. 13

FIG. 14

Europäisches
Patentamt

European
Patent Office

Office européen
des brevets

# EUROPEAN SEARCH REPORT

Application Number

EP 11 15 5219

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/28429 A (ALLECURE CORPORATION; UNIVERSITY OF ZURICH; KUNDIG, THOMAS, M; MCCORMA) 11 April 2002 (2002-04-11) * paragraphs [0063], [0100] - [0103]; claims 1,21 * | 1-15 | INV. A61K39/00 |
| X | VON BOEHMER ET AL.: "The manipulation of immunity", EMBO REPORTS, vol. 5, no. 8, August 2004 (2004-08), pages 766-771, XP002390534, ISSN: 1469-221X * page 770, column 1, paragraph 3 * | 1-15 | |

TECHNICAL FIELDS
SEARCHED       (IPC)

A61K

The present search report has been drawn up for all claims

| Place of search | Date of completion of the search | Examiner |
|---|---|---|
| Munich | 19 May 2011 | Schwachtgen, J |

EPO FORM 1503 03.82 (P04C01)

**ANNEX TO THE EUROPEAN SEARCH REPORT
ON EUROPEAN PATENT APPLICATION NO.**

EP 11 15 5219

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2011

| Patent document cited in search report | | Publication date | Patent family member(s) | | Publication date |
|---|---|---|---|---|---|
| WO 0228429 | A | 11-04-2002 | AU | 9658701 A | 15-04-2002 |
| | | | AU | 2001296587 B2 | 31-08-2006 |
| | | | CA | 2425740 A1 | 11-04-2002 |
| | | | DE | 60125961 T2 | 11-10-2007 |
| | | | DK | 1322330 T3 | 14-05-2007 |
| | | | EP | 1322330 A2 | 02-07-2003 |
| | | | ES | 2281447 T3 | 01-10-2007 |
| | | | PT | 1322330 E | 30-04-2007 |
| | | | US | 2002061315 A1 | 23-05-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 64072704 P
- US 11793702 A
- US 10657022 B
- US 11067159 B
- US 20050221440 A
- US 11067064 B
- US 20050142144 A
- US 10871708 B
- US 60580969 B
- US 581001 P
- US 60580962 P
- US 58100104 P
- US 11156253 P
- US 58096204 P
- US 11155929 P
- US 99918601 A
- US 64040204 P
- US 64082104 P
- US 56046500 A
- US 10026066 A
- US 20030215425 A1
- US 10005905 B
- US 10895523 B
- US 20050130920 A
- US 10896325 B
- US 6861234 B
- US 95640104 A
- US 20050069982 A1
- US 56157100 A
- US 10094699 B
- US 20030046714 A1
- US 11073347 B
- US 10117937 B
- US 20030220239 A1
- US 20040180354 A1

- US 2003027706 W
- WO 04022709 A2
- US 56157200 A
- US 10225568 A
- US 20030138808 A
- US 29241302 A
- US 20030228634 A1
- US 10777053 B
- US 20040132088 A
- US 10837217 B
- US 10225568 B
- US 2003026231 W
- WO 2004018666 A
- US 6709844 B
- US 43783003 A
- US 20030180949 A
- US 47955403 P
- US 87170804 A
- US 2004019571 W
- US 64059804 P
- US 380534 A
- US 09776232 A
- US 20020007173 A1
- US 6977074 B
- US 9814289 W
- WO 9902183 A2
- US 87170704 A
- US 6773695 B
- US 58096404 P
- US 15592805 A
- US 15528805 A
- US 11156369 B
- US 60691889 B
- US 60691579 B
- US 60691581 B

**Non-patent literature cited in the description**

- **WEBER RW.** *Curr Opin Allergy Clin Immunol.,* 2004, vol. 4, 277-83
- **HEIKENWALDER M et al.** *Nat Med.,* 2004, vol. 10 (2), 187-92
- **ATKINS, MB et al.** *Journal of Clinical Oncology,* 1999, vol. 17 (7), 2105
- **M. COLOMBO ; G. TRINCHIERI.** *Cytokine & Growth Factor Reviews,* 2002, vol. 13 (2), 155-168
- **OKAMOTO et al.** *Journal of the National Cancer Institute,* 2003, vol. 95, 316-326

- **OKAMOTO et al.** *Clinical and Diagnostic Laboratory Immunology,* 2004, vol. 11, 483-495
- **MARCIANI, D.J.** *Drug Discovery Today,* 2003, vol. 8, 934-943
- **SADAO et al.** Locoregional immunotheraphy-Topics at the 13th and 14th Meeting of the Japanese Research Society for Surgical Cancer Immunology. *Biotherapy,* 1995, vol. 9 (7), 845-851

- **THOMA-USZYNSKI, S. et al.** Induction of direct antimicrobial activity through mammalian toll-like receptors. *Science,* 2001, vol. 291, 1544-1547
- **AKIRA, S.** Mammalian Toll-like receptors. *Curr Opin Immunol,* 2003, vol. 15, 5-11
- **AHMAD-NEJAD, P. et al.** *Eur J Immunol,* 2002, vol. 32, 1958-1968
- **CHAMAILLARD, M. et al.** *Nat Immunol,* 2003, vol. 4, 702-707
- **MCSORLEY, S. J. et al.** *J Immunol,* 2002, vol. 169, 3914-3919
- **KRIEG, A. M.** *Annu Rev Immunol,* 2002, vol. 20, 709-760
- **HEIL, F. et al.** *Science,* 2004, vol. 303, 1526-1529
- **DIEBOLD, S. S. et al.** *Science,* 2004, vol. 303, 1529-1531
- **WEIGEL, B. J. et al.** *Clin Cancer Res,* 2003, vol. 9, 3105-3114
- **VERTHELYI, D. et al.** *Aids,* 2004, vol. 18, 1003-1008
- **STORNI, T. et al.** *J Immunol,* 2004, vol. 172, 1777-1785
- **HEMMI, H. et al.** *Nat Immunol,* 2002, vol. 3, 196-200
- **DUMMER, R. et al.** *Dermatology,* 2003, vol. 207, 116-118
- **KADOWAKI, N. et al.** *J Exp Med.,* 2001, vol. 194, 863-9
- **BRUGNOLO, F. et al.** *J Allergy Clin Immunol,* 2003, vol. 111, 380-8
- Remington's Pharmaceutical Sciences. Mack Publishing Co, 1990
- Remington's Pharmaceutical Sciences. Mack Publishing Company
- **FINGL et al.** The Pharmacological Basis of Therapeutics. 1975, 1
- **SCHMIDT, U. ; H. WAGNER ; T. MIETHKE.** CpG-DNA upregulates the major acute-phase proteins SAA and SAP. *Cell Microbiol,* 1999, vol. 1, 61
- **SPARWASSER, T., L. et al.** Immunostimulatory CpG-oligodeoxynucleotides cause extramedullary murine hemopoiesis. *J Immunol,* 1999, vol. 162, 2368
- **PASCOLO, S. et al.** *J Exp Med.,* 1997, vol. 185, 2043-2051
- **KRIEG, A. M.** CpG motifs in bacterial DNA and their immune effects. *Annu Rev Immunol,* 2002, vol. 20, 709
- **WITT, P.L. et al.** *Cancer Res.,* 1993, vol. 53 (21), 5176-80
- **CAMPANELLI, A. et al.** *J Am Acad Dermatol.,* 2005, vol. 52 (1), E1